(19) Europäisches Patentamt
European Patent Office
Office européen des brevets

(11) **EP 4 331 592 A1**

(12) **EUROPEAN PATENT APPLICATION**
published in accordance with Art. 153(4) EPC

(43) Date of publication:
**06.03.2024 Bulletin 2024/10**

(21) Application number: **22807560.2**

(22) Date of filing: **13.05.2022**

(51) International Patent Classification (IPC):
*A61K 35/15* (2015.01)   *A61K 35/51* (2015.01)
*A61P 9/10* (2006.01)   *A61P 9/14* (2006.01)
*A61P 43/00* (2006.01)   *C12N 5/071* (2010.01)

(52) Cooperative Patent Classification (CPC):
**A61K 35/15; A61K 35/51; A61P 9/10; A61P 9/14;
A61P 43/00;** C12N 5/06

(86) International application number:
**PCT/JP2022/020222**

(87) International publication number:
**WO 2022/239862 (17.11.2022 Gazette 2022/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.05.2021 JP 2021081426**

(71) Applicants:
• **Stemmed Inc.**
**Kobe-shi, Hyogo, 650-0047 (JP)**
• **Human Life Cord Japan Inc.**
**Tokyo 103-0012 (JP)**

• **Tokushukai Medical Corporation
Osaka-shi, Osaka, 530-0001 (JP)**

(72) Inventors:
• **ASAHARA Takayuki
Kobe-shi, Hyogo 650-0047 (JP)**
• **SALYBEKOV Amankeldi A.
Kamakura-shi, Kanagawa 247-8533 (JP)**
• **KOBAYASHI Shuzo
Kamakura-shi, Kanagawa 247-8533 (JP)**

(74) Representative: **J A Kemp LLP
80 Turnmill Street
London EC1M 5QU (GB)**

(54) **COMPOSITION AND USE THEREOF**

(57)     A cell-derived extracellular vesicle having bioactivities with reduced transplant rejection. A composition includes an extracellular vesicle of a regenerative cell population induced from a biological sample-derived mononuclear cell or a culture thereof.

FIG. 5A

FIG. 5B

EP 4 331 592 A1

**Description**

TECHNICAL FIELD

The present invention relates to a composition and use thereof.

BACKGROUND ART

In various diseases such as inflammatory diseases, ischemic diseases, and the like, treatment using cells has been attempted. For the cells, autologous cells are used. However, when autologous cells are used, the cells need to be obtained from a patient, processed, and administered to the patient, and therefore, such use can take time and processing may vary among cells. For this reason, treatment with allogeneic cells has also been attempted (Patent Literature 1).

Citation List

Patent Literature

Patent Literature 1: JP 2011-529957 A

SUMMARY OF INVENTION

Technical Problem

However, when the allogeneic cells are transplanted into the patient, an immune reaction (transplant rejection) against the allogeneic cells is caused after the transplantation, the allogeneic cells are eliminated from the patient, and a sufficient therapeutic effect cannot be obtained.
Accordingly, an object of the present invention is to provide a cell-derived extracellular vesicle having bioactivities with reduced transplant rejection.

Solution to Problem

In order to achieve the above object, the present invention provides a composition including an extracellular vesicle of a regenerative cell population induced from a biological sample-derived mononuclear cell or a culture thereof.
The present invention also provides a composition for use in treating ischemic heart disease, wherein the composition is the composition according to the preset invention.
The present invention also provides a composition for use in promoting vascular endothelial cell growth, wherein the composition is the composition according to the present invention.
The present invention also provides a composition for use in inducing angiogenesis, wherein the composition is the composition according to the present invention.
The present invention also provides a composition for use in suppressing fibrosis, wherein the composition is the composition according to the present invention.

Advantageous Effects of Invention

According to the present invention, it is possible to provide an extracellular vesicle having bioactivities with reduced transplant rejection.

BRIEF DESCRIPTION OF DRAWINGS

[FIGs. 1A to 1C] FIGs. 1A to 1C are graphs showing the particle size, the production amount per cell, and the mean diameter of the extracellular vesicles in Example 1.
[FIG. 2] FIG. 2 is an electron-microscopic image showing the structure of an RAC-derived extracellular vesicle in Example 1.
[FIGs. 3A and 3B] FIGs. 3A and 3B are histograms each showing the expression of marker in Example 1.
[FIG. 4] FIG. 4 is a graph showing the HUVEC cell growth in Example 1.

[FIGs. 5A and 5B] FIGs. 5A and 5B are photographs and a graph showing the growth area of HUVEC cell in Example 1.

[FIG. 6] FIG. 6 is a graph showing the expression level of miRNA in Example 1.

[FIGs. 7A to 7E] FIGs. 7A to 7E are photographs each showing the echocardiogram in Example 1 and graphs showing the ejection fraction and the left ventricular fractional shortening in Example 1.

[FIGs. 8A and 8B] FIGs. 8A and 8B are graphs showing the mitral regurgitation severity grade and the left ventricular end-systolic volume in Example 1.

[FIGs. 9A and 9B] FIGs. 9A and 9B are a graph and photographs showing the degree of fibrosis in Example 1.

[FIG. 10] FIG. 10 is a graph showing the expression level of miRNA in Example 1.

[FIGs. 11A and 11B] FIGs. 11A and 11B are photographs each showing the blood vessel in infarcted myocardial tissue in Example 1 and a graph.

[FIG. 12] FIG. 12 is a graph showing the expression level of miRNA in Example 1.

[FIG. 13] FIG. 13 shows photographs each showing the fluorescent image of infarcted myocardial tissue in Example 1.

[FIGs. 14A and 14B] FIGs. 14A and 14B are photographs each showing the luminescence intensity of infarcted myocardial tissue and a graph.

[FIGs. 15A and 15B] FIGs. 15A and 15B are graphs showing the body weight and the spleen weight of rat in Example 1.

[FIG. 16] FIG. 16 shows graphs showing the proportion of CD3 positive cells, CD4 positive cells, CD8 positive cells, regulatory T cells, and CD11b/c positive cells in Example 1.

[FIG. 17] FIG. 17 shows graphs showing the proportion of CD3 positive cells, CD4 positive cells, CD8 positive cells, TNK, iNK, regulatory T cells, and CD11b/c positive cells in Example 1.

[FIG. 18] FIG. 18 is a graph showing the expression level of miRNA in Example 1.

[FIGs. 19A and 19B] FIGs. 19A and 19B are graphs each showing the number of cells in Example 2.

[FIGs. 20A and 20B] FIGs. 20A and 20B are graphs each showing the result of EPC-CFA assay in Example 2.

[FIGs. 21A to 21E] FIGs. 21A to 21E are graphs each showing the expression level of miRNA in Example 3.

[FIGs. 22A to 22E] FIGs. 22A to 22E are graphs each showing the expression level of miRNA in Example 3.

DESCRIPTION OF EMBODIMENTS

<Definition>

As used herein, the term "biological sample" refers to a sample derived from a living body, from which cells constituting the living body can be extracted or isolated.

As used herein, the term "mononuclear cell" refers to a cell having a circular nucleus included in peripheral blood, bone marrow, umbilical cord blood, or the like. Examples of the mononuclear cell include lymphocytes, monocytes, macrophages, endothelial progenitor cells, and hematopoietic stem cells. The mononuclear cells may further include CD34 positive cells and/or CD133 positive cells.

As used herein, the term "extracellular vesicle" refers to a vesicle having a membrane secreted from a cell. It is generally thought that the extracellular vesicle is formed within the endosome of the cell of origin and then released out of the cell. Thus, the extracellular vesicle usually includes a lipid bilayer membrane and a lumen, and the lumen is surrounded by the lipid bilayer membrane. The lipid bilayer includes lipid derived from the cell membrane of the cell of origin. The lumen includes a cytoplasm derived from the cell of origin. The extracellular vesicles are classified into, for example, exosome, microvesicle (MV), apoptotic body, and the like according to their size and/or surface-marker.

As used herein, the term "regenerative cell population" refers to a cell population including cells having a regenerative function. The regenerative function means a function of repairing or improving the function of a tissue or an organ, and the regenerative cell population may have any one of these functions. As a specific example, the regenerative function may be vascular endothelial cell growth promoting activity and/or angiogenesis promoting activity.

As used herein, the term "cell population" refers to a cell preparation including desired cells or a composition including desired cells. Thus, in the following description, the cell population can also be referred to, for example, as a cell preparation or a composition. In the cell population, the proportion of the desired cells in all the cells (hereinafter, also referred to as "purity") can be quantified, for example, as the proportion of the desired cells expressing one or more markers. The purity is, for example, the proportion in living cells. The purity can be measured by, for example, flow cytometry, immunohistochemical methods, in situ hybridization, or the like.

In the following description, the purity of the cell preparation is, for example, 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more.

As used herein, the term "culture" refers to cells obtained by culturing cells of a subject and/or a medium of the cells.

As used herein, the term "umbilical cord" refers to a white tubular tissue that connects the fetus and the placenta. In the present invention, the origin of the "umbilical cord" is not particularly limited, and examples thereof include mammals such as mice, rats, rabbits, dogs, cats, cows, horses, pigs, monkeys, dolphins, and sea lions. The origin of the "umbilical cord" is preferably a primate mammal, and more preferably a human.

As used herein, the term "umbilical cord blood" refers to fetus-derived blood contained in the umbilical cord.

As used herein, the term "miRNA" refers to a non-coding RNA having a base length of about 18 to about 25. The miRNA may also be referred to as "micro RNA", "miR", or the like.

As used herein, the term "miRNA precursor" refers to a transcript originating from genomic DNA and including a non-coding RNA having one or more miRNA sequences. The miRNA precursor can also be referred to as "pri-miRNA" or "pre-miRNA". The miRNA precursor usually has a hairpin structure and has a base sequence of a mature miRNA.

As used herein, the term "mir-X" refers to a miRNA precursor of number X, and the term "miR-X" refers to a mature form of miRNA of number X (miRNA). In the following description, when two miRNAs originate from opposing arms of the same miRNA precursor, the two miRNAs are indicated using the suffix "-3p" or "-5p". Also, in the following description, when a miRNA precursor includes two miRNAs, the "miR-X" refers to miRNAs of both -3p and -5p, unless otherwise indicated. Also, in the following description, when the relative expression levels of two miRNAs are known, and the expression level of one miRNA is lower than the expression level of the other miRNA, the miRNA having the lower expression level is indicated as the name with an asterisk. For the information of the base sequences of "mir-X" and "miR-X", reference can be made to miRBase (http://www.mirbase.org/).

As used herein, the expression "promoting cell growth" means that cell growth of a subject is significantly promoted. The cell of a subject is a desired cell. The promoting can also be referred to as improving, enhancing, or increasing. As a specific example, "promoting cell growth " means that the cell growth of a subject is significantly promoted in a treatment group in which a test substance is treated as compared to a control group in which a test substance is not treated or a control group in which a test substance is treated with a substance having no cell growth promoting activity. Therefore, in the present invention, it can be said that "cell growth is promoted" if the cell growth of a subject is significantly promoted as compared to the control group, even if the cell growth activity in the control group is lowered as compared to that at the start of the test. The cell growth activity can be measured, for example, according to Example 1 described below.

As used herein, the expression "promoting angiogenesis" means that angiogenesis or vasculogenesis is significantly promoted. The promoting can also be referred to as improving, enhancing, or increasing. As a specific example, "promoting angiogenesis" means that the angiogenesis or vasculogenesis is significantly promoted in a treatment group in which a test substance is treated as compared to that of a control group in which a test substance is not treated or that of a control group in which a test substance is treated with a substance having no angiogenesis promoting activity. Therefore, in the present invention, it can be said that "angiogenesis is promoted" if the angiogenesis or the hyperplasia amount of the blood vessel is significantly promoted as compared to that of the control group even if the angiogenesis or the vasculogenesis amount is lowered as compared to that at the start of the test. The angiogenesis promoting activity can be measured, for example, according to Example 1 described below.

As used herein, the expression "suppressing fibrosis" means that fibrosis of a subject is significantly suppressed. The term "suppressing" can also be referred to as reducing, lowering, stopping, inhibiting, or preventing. As a specific example, "suppressing fibrosis" means that the fibrosis of the subject is significantly suppressed in a treatment group in which a test substance is treated as compared to that of a control group in which a test substance is not treated or that of a control group in which a test substance is treated with a substance having no fibrosis suppressing activity. Therefore, in the present invention, it can be said "fibrosis inhibition" if fibrosis of the subject is significantly suppressed as compared to that of the control group, even if fibrosis is already inhibited in the control group compared to that at the start of the test in the control group. The fibrosis suppressing activity can be measured, for example, according to Example 1 described below.

As used herein, the term "transplant rejection" or "immune rejection" refers to a host reaction of eliminating transplants caused in a host due to differences in major histocompatibility antigens in the transplantation of cells, tissues, or organs. The transplant rejection may also be referred to as "allotransplant rejection" in the case of allogeneic transplantation. The transplant rejection may also be referred to as "xenotransplant rejection" in the case of xenogeneic transplantation. The "transplant rejection" can be measured, for example, according to Example 1 described below.

As used herein, the expression "suppressing transplant rejection" or "suppressing immune rejection" means that transplant rejection or immune rejection is significantly suppressed. The term "suppressing" can also be referred to as reducing, lowering, stopping, inhibiting, or preventing. The expression "suppressing transplant rejection" means that the transplant rejection is significantly suppressed in a treatment group in which a test substance is treated as compared to that of a control group in which a test substance is treated with a substance inducing the transplant rejection. As a specific example, in the case of examining the transplant rejection of the extracellular vesicles, when the transplant rejection is significantly suppressed in the treatment group of the extracellular vesicles as compared to that of the control group treated with cells from which the extracellular vesicles are derived, it can be said that the transplant rejection of the extracellular vesicles is suppressed. The "transplant rejection" can be measured, for example, according to Example 1 described below.

As used herein, the term "extraction" refers to a state in which a specific component is more concentrated than before extraction. Thus, the extraction can also be referred to as condensation or enrichment. The "extraction" can be carried out, for example, by performing at least one concentration process.

As used herein, the term "isolation" refers to a state in which a specific component is identified and separated and/or recovered from a component in its natural state. The "isolation" can be carried out, for example, by performing at least one purification process.

As used herein, the term "positive (+)" means that the signal detected by an analytical method such as flow cytometry of detecting using an antigen-antibody reaction is higher than that of a negative control reaction using a negative control cell that does not express the antigen or an antibody that does not react with the antigen.

As used herein, the term "negative (-)" means that the signal detected is equivalent to or lower than that of a negative control reaction using a negative control cell that does not express the antigen or an antibody that does not react with the antigen.

As used herein, the term "treatment" refers to therapeutic treatment and/or prophylactic treatment. As used herein, "treatment" means the treatment, cure, prevention, suppression, amelioration, or improvement of a disease, condition, or disorder, or stop, suppression, reduction, or delay of the progression of a disease, condition, or disorder. As used herein, "prevention" refers to a decrease in the possibility of developing a disease or condition, or a delay in the offset of a disease or condition. The term "treatment" may be, for example, treatment for a patient who develops a target disease, or treatment of a model animal with a target disease.

As used herein, the term "effective dose" refers to an amount that, when administered to a patient with a disease, is sufficient to provide a therapeutic effect on the disease.

As used herein, the term "subject" refers to animals or cells, tissues, or organs derived from animals, and is used in a sense that includes, in particular, humans. The animal means human and non-human animals. Examples of the non-human animal include mammals such as mice, rats, rabbits, dogs, cats, cows, horses, pigs, monkeys, dolphins, and sea lions.

Hereinafter, the present invention will be described with reference to examples. However, the present invention is not limited to the following examples and the like, and can be changed and implemented at one's discretion. In addition, the descriptions of the respective embodiments can be referred to each other unless otherwise specified. In the following description, the expression "** to **" is used to include numerical values or physical values before and after "to". Also, in the following description, the expression "A and/or B" includes "A only", "B only", and "both A and B".

<Composition>

In one aspect, the present invention provides a cell-derived extracellular vesicle having bioactivities with reduced transplant rejection, or a composition including the same. The extracellular vesicle of the present invention is an extracellular vesicle of a regenerative cell population induced from a mononuclear cell derived from a biological sample or a culture thereof. The composition of the present invention includes an extracellular vesicle of a regenerative cell population induced from a mononuclear cell derived from a biological sample or a culture thereof. Since the transplant rejection of the extracellular vesicle or composition of the present invention is suppressed, and the extracellular vesicle or composition of the present invention is derived from the regenerative cell population, the extracellular vesicle or composition of the present invention has bioactivities such as vascular endothelial cell growth promoting activity and angiogenesis promoting activity. Thus, the extracellular vesicle or composition of the present invention can be suitably used, for example, for treating or improving the prognosis of ischemic heart disease, as described below.

The regenerative cell population is a cell population induced from mononuclear cells derived from the biological sample. The regenerative cell population includes, for example, cells having a regenerative function that can be induced from the mononuclear cells. Examples of the cells having a regenerative function include endothelial progenitor cells and anti-inflammatory macrophages, and may include Th2 cells and regulatory T cells (Treg). The endothelial progenitor cells and the anti-inflammatory macrophages have, for example, angiogenesis promoting activity and anti-inflammatory activity as the regenerative function, and thus are extremely useful in not only angiogenesis but also in suppression of inflammation at inflammatory sites such as ulcers. The regenerative cell population includes one or more types of cells having a regenerative function.

The endothelial progenitor cells (EPCs) are cells capable of differentiating into vascular endothelial cells. The endothelial progenitor cells can be identified using cell-surface markers such as CD34, CD133, KDR (kinase insert domain receptor, CD309), CD105, and the like.

The endothelial progenitor cells may be further classified according to the degree of differentiation. In this case, the endothelial progenitor cells can be further classified into differentiated EPC colony cells and undifferentiated EPC colony cells. The differentiated EPC colony cells mainly include cells having a diameter of 20 to 50 $\mu$m (CFU-large cell like EC, hereinafter also referred to as a "large EPC colony"). The undifferentiated EPC colony cells mainly include cells having a diameter of 20 $\mu$m or less (CFU-small cell like EC, hereinafter also referred to as a "small EPC colony"). As to the differentiation from the endothelial progenitor cells to vascular endothelial cells, it can be said that a small EPC colony that appears at an early stage is an EPC colony of early differentiation stage that has excellent growth, while a large EPC colony that appears at a later stage is a colony of late differentiation stage that has excellent angiogenesis (see, e.g., Masuda H. et al., Circulation Research, 109: 20-37 (2011)). Since the differentiated EPC colony cells are, for example, superior to the undifferentiated EPC colony cells in regenerative function such as angiogenesis or repairing, the endothelial progenitor cells are preferably differentiated EPC colony-forming cells.

The anti-inflammatory macrophage is a type of macrophage that secretes anti-inflammatory cytokines (IL-10, IL-4) and the like, and is a cell that contributes to angiogenesis or repairing. The anti-inflammatory macrophage can be identified, for example, using cell-surface markers such as CD206, CD163, CD169, and the like. The anti-inflammatory macrophages are preferably M2 macrophages, for example, because they have an excellent regenerative function such as angiogenesis or repairing.

The proportion of the cells having a regenerative function in all cells of the regenerative cell population is not particularly limited, and is, for example, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 96% or more, 97% or more, 98% or more, or 99% or more. The proportion of the cells can be calculated, for example, by measuring the proportion of intended cell-surface marker-positive cells using the cell-surface marker having a regenerative function. The measurement can be performed using, for example, flow cytometry.

The proportion of the cells having a regenerative function in all cells of the regenerative cell population is usually increased by a factor of two, preferably by a factor of four or more, as compared to, for example, the proportion of cells having a corresponding regenerative function in mononuclear cells derived from the biological sample.

When the cells having a regenerative function are the endothelial progenitor cells and the anti-inflammatory macrophages, the proportion of the cells having a regenerative function in all cells of the regenerative cell population can be calculated, for example, as the sum of the proportion of CD34 positive cells and the proportion of CD206 positive cells.

In the regenerative cell population, the cell having the regenerative function is induced from the mononuclear cell. Therefore, the biological sample used for inducing the regenerative cell population may be a biological sample containing the mononuclear cells. The mononuclear cells are rich in blood and lymph. Thus, the biological sample may be a tissue or an organ including blood vessels and/or lymphatic vessels, or may be blood and/or a lymphatic fluid. Specific examples of the biological sample include bone marrow; blood such as umbilical cord blood, peripheral blood, and the like; and lymph.

The biological sample may be a biological sample collected from a subject to be administered, treated, or medically cared with the extracellular vesicle and/or composition of the present invention (hereinafter, collectively referred to as "subject to be administered"), or may be a biological sample collected from a subject other than the subject to be administered. From the viewpoint of being not limited in preparation, it is desirable to use a biological sample collected from a subject other than the subject to be administered. The regenerative cell population may be cells (heterogeneous cells) derived from a biological sample other than a subject to be administered or cells (autologous cells) derived from a biological sample collected from a subject to be administered, as will be described in the Examples below. In the present invention, since the transplant rejection of the extracellular vesicles is suppressed, the extracellular vesicles can be suitably prepared even using a biological sample collected from a subject other than the subject to be administered. In addition, it has been confirmed that the extracellular vesicles have a therapeutic effect without being eliminated by, for example, immune rejection. Thus, the extracellular vesicles can be prepared, for example, as an off-the-shelf formulation.

The method for preparing the biological sample is not particularly limited, and can be appropriately determined according to, for example, the type of the biological sample. As a specific example, when the biological sample is bone marrow, the biological sample can be obtained, for example, by performing bone marrow aspiration on the subject. When the biological sample is peripheral blood, the biological sample can be obtained by, for example, collecting venous blood. When the biological sample is umbilical cord blood, the biological sample can be performed, for example, by isolating the umbilical cord and collecting blood in the umbilical cord.

The method for separating mononuclear cells from the biological sample can be performed according to a method commonly performed in the art, for example, by separating the mononuclear cells and other components using a cell suspension prepared from the biological sample and a mononuclear cell separation solution such as Ficoll or Percoll.

A method for inducing the cells having the regenerative function from mononuclear cells derived from the biological sample can be performed by a known method according to, for example, the type of cells having a regenerative function. Specifically, when endothelial progenitor cells are induced from the mononuclear cells, the inducing methods can be performed by culturing the mononuclear cells in the presence of, for example, SCF, IL-6, FLT3L, TPO, and/or VEGF. In addition, when anti-inflammatory macrophages are induced from the mononuclear cells, the inducing method can be performed by culturing the mononuclear cells in the presence of, for example, IL-10, IL-4, M-CSF and/or G-CSF.

The inducing method is preferably a method capable of inducing both the endothelial progenitor cells and the anti-inflammatory macrophages from the mononuclear cells. For the inducing method, for example, reference can be made to the inducing method described in WO 2014/051154, which is herein incorporated by reference.

Since the regenerative cell population can induce the cells having a regenerative function including, for example, the extracellular vesicles having angiogenic activity and anti-inflammatory activity, it is preferable that the mononuclear cells be cultured in the presence of factors such as a stem cell factor (SCF), interleukin-6 (IL-6), FMS like tyrosine kinase-3 ligand (Flt3L), thrombopoietin (TPO), and/or a vascular endothelial cell growth factor (VEGF). The culturing can be performed using a medium containing the factor.

As the mononuclear cells to be subjected to the culturing, a desired cell population obtained by sorting a desired cell population from mononuclear cells derived from the biological sample may be used, or mononuclear cells derived from the biological sample without being sorted may be used. As the mononuclear cells to be subjected to the culturing, it is preferable to use mononuclear cells (non-sorted mononuclear cells) obtained without sorting CD34 positive cells and/or CD133 positive cells. From this, the regenerative cell population obtained after the culturing can be, for example, a cell population in which the differentiated EPC colony-forming cells are increased (amplified), the anti-inflammatory macrophages are increased (amplified), and the anti-inflammatory Th2 cells and the regulatory T cells are increased (amplified), as compared to, for example, those of where mononuclear cells sorted as CD34 positive cells are used. Therefore, by using the non-sorted mononuclear cells, for example, a cell population in which the proportion of the cells having a regenerative function is enriched can be obtained as the regenerative cell population, thereby obtaining extracellular vesicles excellent in bioactivities such as vascular endothelial cell growth promoting activity, angiogenesis promoting activity, fibrosis suppressing activity, transplant rejection suppressing activity, and immunosuppressive activity.

The SCF is a glycoprotein of about 30,000 molecular weight consisting of 248 amino acids. Alternative splicing of the SCF results in soluble SCF or membrane-bound SCF. The SCF for use in culturing can be either type of SCF as long as it is useful for culturing a regenerative cell population such as EPC. The SCF is preferably soluble SCF. The origin or the like of SCF is not particularly limited, and is preferably a recombinant, particularly preferably a human recombinant because a stable feed is expected. The human recombinant SCF is commercially available. The concentration of SCF in the medium can be determined according to the type of SCF, and is not particularly limited as long as it is useful for culturing a regenerative cell population such as EPC. As a specific example, when SCF is a human recombinant SCF, the concentration of SCF in the medium is, for example, 10 to 1000 ng/ml, preferably 50 to 500 ng/ml, and more preferably about 100 ng/ml.

The IL-6 is a glycoprotein having a molecular weight of about 21,000 isolated as a factor for inducing terminal differentiation of B cells into antibody-producing cells, and is known to be involved in immune responses, growth differentiation of hematopoietic and nervous system cells, acute-phase reactions, and the like. The origin or the like of the IL-6 is not particularly limited, and is preferably a recombinant, particularly preferably a human recombinant because a stable feed is expected. The human recombinant IL-6 is commercially available. The concentration of IL-6 in the medium varies depending on the type of IL-6 used, and is not particularly limited as long as it is useful for culturing a regenerative cell population such as EPC. As a specific example, when IL-6 is human recombinant IL-6, the concentration of IL-6 in the medium is, for example, 1 to 500 ng/ml, preferably 5 to 100 ng/ml, and more preferably about 20 ng/ml.

The Flt3L is known as a ligand for a receptor tyrosine kinase, which plays a key role in early hematopoietic control. As to the Flt3L, although several products that depend on alternative splicing are known, it has been reported that the Flt3L stimulates the growth of hematopoietic stem cells. The Flt3L may be any type of Flt3L as long as it is useful for culturing a regenerative cell population, such as EPC. The origin or the like of Flt3L is not particularly limited, and is preferably a recombinant, particularly preferably a human recombinant because a stable feed is expected. The human recombinant Flt3L is commercially available. The concentration of Flt3L in the medium varies depending on the type of Flt3L used, and is not particularly limited as long as it is useful for culturing a regenerative cell population such as EPC. As a specific example, when Flt3L is a human recombinant Flt3L, the concentration of Flt3L in the medium is, for example, 10 to 1000 ng/ml, preferably 50 to 500 ng/ml, and more preferably about 100 ng/ml.

The TPO is a type of hematopoietic cytokine and is known to specifically act in a process of producing megakaryocytes from hematopoietic stem cells, thereby promoting the production of megakaryocytes. The origin or the like of TPO is not particularly limited, and is preferably a recombinant, particularly preferably a human recombinant because a stable feed is expected. The human recombinant TPO is commercially available. The concentration of TPO in the medium varies depending on the type of TPO used, and is not particularly limited as long as it is useful for culturing a regenerative cell population such as EPC. As a specific example, when TPO is a human recombinant TPO, the concentration of TPO in the medium is, for example, 1 to 500 ng/ml, preferably 5 to 100 ng/ml, and more preferably about 20 ng/ml.

The VEGF is a growth factor that acts specifically on EPC and is known to be produced primarily in perivascular cells. Alternative splicing of the VEGF results in several types of VEGF proteins of different sizes. The VEGF may be any type of VEGF as long as it is capable of colonizing EPC contained in the regenerative cell population, and is preferably VEGF 165. The origin or the like of VEGF is not particularly limited, and is preferably a recombinant, particularly preferably a human recombinant because a stable feed is expected. The human recombinant VEGF is commercially available. The concentration of VEGF in the medium varies depending on the type of VEGF used, and is not particularly limited as long as it is useful for culturing a regenerative cell population such as EPC. When VEGF is a human recombinant VEGF 165, the concentration of VEGF in the medium is, for example, about 5 to 500 ng/ml, preferably about 20 to 100 ng/ml, more preferably about 50 ng/ml.

As the various factors to be added to the medium, factors derived from animals of the same species as or different species from the animal from which the mononuclear cells are derived are used, and it is preferable to use factors derived from animals of the same species. By unifying the origin of the mononuclear cells and various factors, a regenerative cell population suitable for allogeneic transplantation such as allotransplantation, and extracellular vesicles derived therefrom are obtained. Furthermore, by using mononuclear cells derived from the subject to be administered, it is also possible to obtain extracellular vesicles suitable for isogeneic transplantation. Thus, by culturing a regenerative cell population containing EPC or the like in an environment that does not contain any components derived from a heterologous animal, the resulting regenerative cell population and the extracellular vesicles derived therefrom can reduce the risk of infection and rejection upon administration or the like.

The medium for use in the culturing may be prepared by dissolving the various factors in a basal medium so as to achieve a predetermined concentration or may be prepared by preparing a concentrate (stock solution) containing the various factors in advance and diluting the concentrate in a desired medium so as to achieve a predetermined concentration. The medium for use in the culturing may be prepared by dissolving various necessary factors in a commercially available basal medium so as to achieve a predetermined concentration, and then sterilizing by filter sterilization or the like, or may be prepared by aseptically adding a stock solution sterilized by filter sterilization or the like to a commercially available basal medium and then diluting. The filter sterilization can be carried out according to a method usually carried out in the art, for example, using a Millipore filter having a pore size of 0.22 $\mu$m or 0.45 $\mu$m.

As the basal medium, a basal medium commonly used in the art can be used. For example, a basal medium known as a medium for hematopoietic stem cell growth can be used. The basal medium may be a serum-containing medium or a medium containing no serum, i.e., a serum-free medium. In the culturing of the regenerative cell population, by using a serum-free medium as the medium and/or the basal medium, in the culturing (induction) of the regenerative cell population from the mononuclear cells, the cells having a regenerative function can be efficiently induced (amplified). Therefore, the medium is preferably a serum-free medium. Examples of the medium or the basal medium include DMEM, MEM, and IMDM.

The medium contains at least one factor selected from, preferably at least three factors selected from, or more preferably all factors of the group consisting of SCF, IL-6, FLT3L, TPO, and VEGF. Therefore, the medium for use in the culturing contains, for example, any of the following:

(1) SCF, (2) IL-6, (3) FLT3L, (4) TPO, or (5) VEGF;
(6) combination of SCF and IL-6, (7) combination of SCF and FLT3L, (8) combination of SCF and TPO, (9) combination of SCF and VEGF, (10) combination of IL-6 and FLT3L, (11) combination of IL-6 and TPO, (12) combination of IL-6 and VEGF, (13) combination of FLT3L and TPO, (14) combination of FLT3L and VEGF, or (15) combination of TPO and VEGF;
(16) combination of SCF, IL-6, and FLT3L, (17) combination of SCF, IL-6, and TPO, (18) combination of SCF, FLT3L, and TPO, (19) combination of SCF, FLT3L, and VEGF, (20) combination of IL-6, FLT3L, and TPO, or (21) combination of IL-6, FLT3L, and VEGF;
(22) combination of SCF, IL-6, FLT3L, and TPO, (23) combination of SCF, IL-6, FLT3L, and VEGF, or (24) combination of IL-6, FLT3L, TPO and VEGF; or
(25) combination of SCF, IL-6, FLT3L, TPO, and VEGF.

More preferably, the medium contains SCF, IL-6, FLT3L, TPO, and VEGF. Still more preferably, the medium contains about 50 ng/mL VEGF, about 100 ng/mL SCF, about 20 ng/mL IL-6, 100 ng/mL FLT3L, and about 20ng/mL TPO.

The culturing of the mononuclear cells using a medium containing the various factors can be carried out by adding a cell suspension containing the mononuclear cells to a medium containing the various factors. As the cell suspension, a body fluid (e.g., bone marrow fluid, umbilical cord blood, peripheral blood) containing the mononuclear cells may be used. The culturing conditions of the mononuclear cells are not particularly limited, and the culturing can be usually carried out under conditions performed in the art. As a specific example, the culturing is carried out at 37°C for 7 days or more (for example, 10 days or more) in, for example, a 5% $CO_2$ atmosphere. The concentration (seeding density) of the mononuclear cells in the medium is not particularly limited as long as it allows culturing of a regenerative cell population such as EPC. The concentration (seeding density) of the mononuclear cells in the medium is, for example, about 0.5 to $10 \times 10^5$ cells/ml, more preferably about 1 to $5 \times 10^5$ cells/ml, and still more preferably about 3 to $4 \times 10^5$ cells/ml.

The culture of the regenerative cell population may be a culture obtained in the induction of the regenerative cell population from the mononuclear cells or may be a culture obtained by culturing the induced regenerative cell population.

The medium and the culturing conditions of the regenerative cell population are not particularly limited, and for example, reference can be made to the description as to the medium and the culturing conditions of the mononuclear cells. As a specific example, as the medium for the regenerative cell population, for example, a medium obtained by adding serum, preferably human-derived serum, to the basal medium may be used. The origin of the serum may be the same as or different from the origin of the regenerative cell population, and is preferably the same as the origin of the regenerative cell population. The serum may contain the extracellular vesicles. Thus, when the serum is added to the basal medium, it is preferable to use the serum from which the extracellular vesicles have been removed. The serum from which the extracellular vesicles have been removed is commercially available. The concentration of the serum is, for example, from 1 to 20 (v/v)%, and preferably from 1 to 10 (v/v)%.

The culturing of the regenerative cell population can be carried out by adding a cell suspension containing the regenerative cell population to a medium of the regenerative cell population. The culturing conditions of the regenerative cell population are not particularly limited, and the culturing can be usually carried out under conditions performed in the art. As a specific example, the culturing is carried out at 37°C for 1 to 10 days (for example, 1 to 2 days) in, for example, a 5% $CO_2$ atmosphere. The concentration (seeding density) of the regenerative cell population in the medium is not particularly limited as long as it allows culturing of the regenerative cell population. The concentration (seeding density) of the regenerative cell population in the medium is, for example, about $1 \times 10^4$ to $1 \times 10^7$ cells/ml, more preferably about $1 \times 10^5$ to $1 \times 10^6$ cells/ml, and still more preferably about $5 \times 10^5$ cells/ml.

The extracellular vesicles are secreted from the cells of origin, as described above. Thus, the extracellular vesicles may be isolated or extracted from the regenerative cell population or a culture thereof.

The extracellular vesicles can be isolated or extracted from the regenerative cell population, for example, by disrupting the regenerative cell population, releasing the extracellular vesicles in the cells of the regenerative cell population, separating a soluble fraction from the obtained suspension, and then subjecting the obtained soluble fraction to a method for isolating or extracting. The method for isolating or extracting the extracellular vesicles can be performed according to a method commonly performed in the art, and can be performed, for example, by a method using centrifugation, an affinity purification method using a membrane protein derived from a regenerative cell population present in a membrane of the extracellular vesicles, or filtration using a porous membrane such as a filter. As the filter, for example, a commercially available membrane filter can be used, and as a specific example, a membrane filter having a pore size of 0.22 μm (manufactured by Millipore Corporation) can be used. The membrane protein of the extracellular vesicle is described below. The extracellular vesicle may be isolated or extracted by using a commercially available kit, and as a specific example, qEv isolation kit (manufactured by Izon Science Limited) can be used.

When the extracellular vesicles are isolated or extracted by the centrifugation, the centrifugation conditions may be any conditions capable of precipitating the extracellular vesicles, and for example, the centrifugation may be performed at 10000 to 300000 × g or 90000 to 180000 × g for 20 to 60 minutes. The temperature during the centrifugation is, for example, 0 to 10°C, preferably 0 to 4°C

The culture is composed, for example, of the regenerative cell population and a medium (conditioned medium) thereof. Thus, when the extracellular vesicles are isolated from the culture, the extracellular vesicles may be isolated or extracted from the regenerative cell population or the conditioned medium, or may be separated from both of the regenerative cell population and the conditioned medium. The method for separating the regenerative cell population and the conditioned medium from the culture can be performed according to a method commonly performed in the art, and can be performed by, for example, filter filtration, centrifugation, or the like. When the regenerative cell population is included, the extracellular vesicles can be isolated or extracted in the same manner as in the case of extracting or isolating the extracellular vesicles from the regenerative cell population described above. When the conditioned medium is used, the method for isolating or extracting the extracellular vesicles can be performed, for example, by a method using centrifugation, an affinity purification method using a membrane protein derived from a regenerative cell population present in a membrane of the extracellular vesicles or the membrane protein, or filtration using a porous membrane such as a filter. The extracellular vesicle may be isolated or extracted by using a commercially available kit, and as a specific example, qEv isolation kit (manufactured by Izon Science Limited) can be used.

Since the extracellular vesicles are derived from the regenerative cell population, the extracellular vesicles contain components derived from the regenerative cell population. The extracellular vesicle has, for example, a lipid bilayer membrane and a lumen, and the lumen is surrounded by the lipid bilayer membrane. In the extracellular vesicle, the lipid bilayer membrane is derived, for example, from the membrane of the regenerative cell population, specifically the membrane of the cell of the regenerative cell population. Examples of the membrane include cell membranes and membranes of intracellular organelles such as mitochondria, endoplasmic reticulum, endosome, lysosome, and Golgi apparatus. In the extracellular vesicle, the lumen has, for example, a cytoplasm derived from the regenerative cell population, specifically, a cytoplasm derived from a cell of the regenerative cell population. Thus, the extracellular vesicles include, for example, miRNAs, DNAs, and/or proteins derived from the cytoplasm of the cells of the regenerative cell population.

The lipid bilayer membrane of the extracellular vesicle includes, for example, a membrane component of cells of the regenerative cell population. Thus, the extracellular vesicle can be defined, for example, using membrane components present in the regenerative cell population. The membrane component may be, for example, a membrane protein. Examples of the membrane protein include extracellular vesicle markers such as CD9, CD63, ALG-2-interacting protein X (Alix), heat shock protein 70 (Hsp-70), and the like; and cell-surface markers of the cells. The cell-surface marker can be appropriately determined according to the type of the cell. When the regenerative cell population includes EPC, the cell-surface marker may be, for example, CD34, CD133, KDR (kinase insert domain receptor, CD309), CD105, or the like. When the regenerative cell population includes the anti-inflammatory macrophage, examples of the cell-surface marker include CD206, CD163, and CD169. The lipid bilayer membrane of the extracellular vesicle includes, for example, one or more membrane components. The membrane component can be measured by flow cytometry using, for example, an antibody against a molecule of a subject.

The lumen of the extracellular vesicle includes, for example, a cytoplasm of cells of the regenerative cell population. Thus, the extracellular vesicle can be defined, for example, using cytoplasmic components present in the regenerative cell population. The cytoplasmic component may be, for example, miRNA. Examples of the miRNA include miR-15b-5p, miR-29b-3p, miR-29c-3p, miR-92a-2-5p, miR-126-3p, miR-126-5p, miR-133a-3p, miR-133b, miR-146a-3p, miR-146b-5p, miR-150-5p, miR-181b-3p, miR-195-3p, miR-195-5p, miR-200b-5p, miR-302a-5p, miR-142-3p, miR-10a-3p, miR-17-3p, miR-20b-5p, miR-21-5p, miR-24-2-5p, miR-29a-5p, miR-30b-5p, miR-30c-5p, miR-30e-3p, miR-30e-5p, miR-32-5p, miR-92a-3p, miR-103a-2-5p, miR-144-5p, miR-148a-3p, miR-199b-5p, miR-200a-3p, miR-205-5p, miR-210-3p, miR-221-5p, miR-324-5p, miR-363-3p, miR-373-3p, miR-509-3p, miR-633, and let-7c-5p. Among the miRNAs, in particular, miR-92a-2-5p, miR-133a-3p, miR-133b, miR-146a-3p, miR-200b-5p, miR-302a-5p, and miR-142-3p are envisaged as specific miRNAs in the extracellular vesicles of the regenerative cell population. Thus, the lumen of the extracellular vesicle preferably includes miR-92a-2-5p, miR-133a-3p, miR-133b, miR-146a-3p, miR-200b-5p, miR-302a-5p, and/or miR-142-3p. The lumen of the extracellular vesicle includes, for example, one or more cytoplasmic components. The composition of the cytoplasm can be measured by flow cytometry using, for example, an antibody against a target molecule. When the cytoplasmic component is a nucleic acid molecule, the cytoplasmic component can be measured using, for example, a sequencer. In the case of a regenerative cell population derived from peripheral blood, the miRNA preferably includes, for example, miR-15b-5p, miR-29b-3p, miR-92a-2-5p, miR-146a-3p, miR-146b-5p, miR-150-5p, miR-195-3p, miR-195-5p, miR-200b-5p, miR-302a-5p, and/or miR-142-3p. In the case of a regenerative cell population derived from umbilical cord blood, the miRNA preferably includes, for example, miR-10a-3p, miR-17-3p, miR-20b-5p, miR-21-5p, miR-24-2-5p, miR-29a-5p, miR-30b-5p, miR-30c-5p, miR-30e-3p, miR-30e-5p, miR-32-5p, miR-92a-3p, miR-103a-2-5p, miR-144-5p, miR-148a-3p, miR-199b-5p, miR-200a-3p, miR-205-5p, miR-210-3p, miR-221-5p, miR-324-5p, miR-363-3p, miR-373-3p, miR-509-3p, miR-633, and/or let-7c-5p.

Among the miRNA, miR-20b-5p, miR-30b-5p, miR-30c-5p, miR-30e-3p, miR-30e-5p, miR-32-5p, miR-92a-2-5p, miR-92a-3p, miR-199b-5p, miR-200a-3p, miR-363-3p, and miR-509-3p are generally known to have anti-apoptotic activity. Among the miRNA, miR-17-3p, miR-20b-5p, miR-103a-2-5p, miR-150-5p, miR-181b-3p, miR-200a-3p, miR-205-5p, miR-302a-5p, miR-324-5p, miR-363-3p, and miR-633 are generally known to have cell growth promoting activity. Among the miRNA, miR-150-5p is generally known to have cell migration promoting activity. Among the miRNA, miR-21-5p, miR-29a-5p, miR-29b-3p, miR-29c-3p, miR-133a-3p, miR-133b, miR-200a-3p, and miR-373-3p are generally known to have anti-fibrotic activity, in particular, TGF-β expression suppressing activity. Among the miRNA, miR-15b-5p, miR-29c-3p, miR-126-3p, miR-126-5p, miR-146a-3p, miR-146b-5p, miR-181b-3p, miR-195-3p, miR-195-5p, miR-200b-5p, miR-210-3p, miR-221-5p, miR-633, and let-7c-5p are generally known to have angiogenesis promoting activity. Among the miRNA, miR-10a-3p, miR-21-5p, miR-24-2-5p, miR-142-3p, miR-144-5p, and miR-148a-3p are generally known to have anti-inflammatory activity.

In the extracellular vesicle, a component contained in the lumen may be replaced with a component other than the cytoplasm of the regenerative cell population. In such a case, the lumen is loaded with a desired cargo. Examples of the cargo include a low molecular weight compound, a protein or a peptide such as an antibody, and a nucleic acid.

The mean diameter (weighted average) of the extracellular vesicles is, for example, 1 to 500 nm, preferably 10 to 250 nm, more preferably 30 to 150 nm. The mean diameter can be measured in accordance with Example 1 described below. The mean diameter of the extracellular vesicles can be adjusted by, for example, filtering a liquid containing the extracellular vesicles using a filter having a desired pore size or the like.

The extracellular vesicle or composition of the present invention may further include a pharmaceutically acceptable carrier. Examples of the carrier include suspensions, dissolve assists, stabilizers, isotonic agents, preservatives, adsorptions, surfactants, diluents, media, pH modifiers, painless agents, buffers, sulfur-containing reducing agents, and antioxidants for administration of the extracellular vesicles. The carrier can be suitably added to the extent that it does not interfere with the effectiveness of the present invention.

The extracellular vesicle or composition of the present invention may be used, for example, *in vitro* or *in vivo*. The extracellular vesicle or composition of the present invention can be used, for example, as a research reagent or as a pharmaceutical.

The subject to which the extracellular vesicle or composition of the present invention is administered is not particularly limited. When the extracellular vesicle or composition of the present invention is used *in vivo,* the subject to be administered may be, for example, a human or a non-human animal other than a human. Examples of the non-human animal include mammals such as mice, rats, rabbits, dogs, cats, cows, horses, pigs, monkeys, dolphins, and sea lions; birds; and fish. When the extracellular vesicle or composition of the present invention is used *in vitro,* the subject to be administered may be, for example, a cell, a tissue, an organ, or the like, and the cell may be, for example, a cell collected from a living body, a cultured cell, or the like, and the tissue or organ may be, for example, a tissue (living tissue) or an organ collected from a living body. Examples of the cell include myocytes, induced pluripotent stem cells (iPS cells), and stem cells.

When the extracellular vesicle or composition of the present invention is used *in vivo,* the subject to be administered may be, for example, a subject diagnosed with or suspected of having ischemic heart disease. Examples of the ischemic heart disease include myocardial infarction, peripheral arterial disease, myocardial ischemia reperfusion injury, and severe leg ischemia.

The use conditions (administration conditions) of the extracellular vesicle or the composition of the present invention are not particularly limited, and for example, the dosage form, the administration timing, the dosage, and the like can be appropriately set according to the type of the subject to be administered, and the like.

Examples of the method for administering the extracellular vesicle or the composition of the present invention include oral administration and parenteral administration such as intravenous administration, and, for example, intravenous administration is preferable because it can be safely and stably administered regardless of the skill of the administrator.

The dosage of the extracellular vesicle or composition of the present invention is an effective dose, and in particular, the dose that, when administered to a subject, can give a therapeutic effect on ischemic heart disease as compared to a subject who has not been administered. Specifically, the dosage can be appropriately determined according to, for example, the age, weight, symptoms, and the like of the subject. Specifically, the dosage may be, for example, $10^4$ to $10^9$ cells/kg weight, $10^4$ to $10^8$ cells/kg weight, or $10^4$ to $10^7$ cells/kg weight per administration as the number of extracellular vesicles, and is preferably $10^4$ to $10^8$ cells/kg weight or $10^4$ to $10^7$ cells/kg weight.

The number of administrations of the extracellular vesicle or composition of the present invention is one or more than one such as 2 times, 3 times, 4 times, 5 times or more. The number of administrations may be determined as appropriate while checking the therapeutic effect on the subject. In the case of the plurality of administrations, the administration interval can be appropriately determined while checking the therapeutic effect on the subject, and the extracellular vesicle or composition of the present invention can be administered once a day, once a week, once every two weeks, once a month, once every three months, or once every six months.

As described above, since the transplant rejection of the extracellular vesicle or composition of the present invention is suppressed and the extracellular vesicle or composition of the present invention is derived from the regenerative cell population, the extracellular vesicle or composition of the present invention has bioactivity such as angiogenesis promoting activity. Thus, the extracellular vesicle or composition of the present invention can be suitably used, for example, for the treatment of ischemic heart disease or for the improvement of the prognosis, as described below.

Use of Extracellular Vesicle or Composition

In another aspect, the present invention provides a composition for use in the treatment of ischemic heart disease or a method using the same. In this case, the present invention is a composition for use in the treatment of ischemic heart disease, wherein the composition is the extracellular vesicle or composition of the present invention. The present invention also is a method for treating a patient with ischemic heart disease, wherein the extracellular vesicle or composition of the present invention is administered to the patient with ischemic heart disease.

Examples of the ischemic disease include myocardial infarction, peripheral arterial disease, myocardial ischemia reperfusion injury, and severe leg ischemia.

In another aspect, the present invention provides a composition for use in promoting vascular endothelial cell growth or a method using the same. In this case, the present invention is a composition for use in promoting vascular endothelial cell growth, wherein the composition is the extracellular vesicle or composition of the present invention. The present invention also is a method for promoting the vascular endothelial cell growth, including promoting the vascular endothelial cell growth by contacting the vascular endothelial cells with a composition, wherein the composition includes an extracellular vesicle or composition of the present invention.

In another aspect, the present invention provides a composition for use in inducing angiogenesis or a method using the same. In this case, the present invention is a composition for use in inducing angiogenesis, wherein the composition is the extracellular vesicle or composition of the present invention. The present invention also is a method for inducing angiogenesis, using the extracellular vesicle or composition of the present invention.

In the method for inducing angiogenesis of the present invention, for example, the extracellular vesicle or the composition is administered to a subject to be induced with angiogenesis. The subject to be induced with angiogenesis is, for example, a patient with ischemic heart disease or the like. In this case, the induction of angiogenesis can also be referred to as induction of angiogenesis in the heart or at a site with ischemia-reperfusion injury (e.g., central or peripheral blood vessel) of the patient.

In another aspect, the present invention provides a composition for use in suppressing fibrosis or a method using the same. In this case, the present invention is a composition for use in suppressing fibrosis, wherein the composition is the extracellular vesicle or composition of the present invention. The present invention also is a method for suppressing fibrosis, using the extracellular vesicle or composition of the present invention.

In the method for suppressing fibrosis of the present invention, for example, the extracellular vesicle or the composition is administered to a subject to be suppressed with fibrosis. The subject to be suppressed with fibrosis may be, for example, a patient with ischemic heart disease or the like. In this case, the induction of angiogenesis can also be referred to as suppression of fibrosis in the heart or at a site with ischemia-reperfusion injury (e.g., central or peripheral blood vessel) of the patient.

Use of Extracellular Vesicle or Composition

The present invention is an extracellular vesicle or composition for use in treating ischemic heart disease. The present invention is an extracellular vesicle or composition for use in promoting vascular endothelial cell growth. The present invention is an extracellular vesicle or composition for use in promoting angiogenesis. The present invention is an extracellular vesicle or composition for use in suppressing fibrosis.

Examples

Hereinafter, the present invention will be described in detail with reference to examples. It is to be noted, however, that the present invention is not limited to the aspect described in the examples. Unless otherwise indicated, commercially available reagents and kits were used according to the protocol.

Example 1

It was examined that the extracellular vesicle of the present invention has vascular endothelial cell growth activity, angiogenesis promoting activity, and fibrosis suppressing activity, with reduced transplant rejection.

(1) Preparation of Regenerative Cell Population

Regenerative cell populations were prepared by inducing from human peripheral blood mononuclear cells. First, 20 to 100 ml of peripheral blood was collected from healthy volunteers aged 20 to 55 years using heparinized alar needles attached to 50 ml syringes. Prior to the collection, informed consent was given to each healthy subject. In addition, the collection was conducted under the approval of the Medical Research Committee of the Tokai University School of Medicine, and the obtained peripheral blood samples were handled in accordance with the biological guidelines for human samples. In addition, the following animal experimentation was conducted with the approval of the Animal Experimentation Committee of the Tokai University School of Medicine in accordance with the guidelines for handling experimental animals (National Research Council).

Isolation of peripheral blood mononuclear cells (PBMNC) from peripheral blood was performed by density gradient centrifugation using Histopaque-1077 (Sigma-Aldrich, Inc., #10771) according to the methods described in Asahara et al., Science, 275: 964-7 (1997) and Amankeldi et.al., PLoSONE, (2019), 14(3): e0205477, which is herein incorporated by reference. The isolated PBMNC was washed with phosphate buffer (PBS)-ethylenediaminetetraacetic acid (EDTA) and then suspended in a serum-free medium (QQ medium) described below, thereby preparing a cell suspension. In the isolated PBMNC, the CD34 positive rate was $0.23 \pm 0.03\%$, and the CD133 positive rate was $0.20 \pm 0.07\%$. The number of CD34 positive cells per peripheral blood (100 ml) was $(27.8 \pm 4.5) \times 10^4$ and the number of CD133 positive cells per peripheral blood (100 ml) was $(23.2 \pm 6.9) \times 10^4$.

The serum-free medium (hereinafter also referred to as "QQ medium" or "QQ cm") for use in culturing (inducing) the regenerative cell population was aseptically added to STEMLINE™ II Hematopoietic Stem Cell Expansion Medium (Sigma-Aldrich, Inc., Cat No. S0192) such that the following factors had predetermined concentrations.

(Concentrations of Factors in QQ Medium)

rhSCF (manufactured by Peprotech, Inc., Cat. No. #300-07): 100 ng/ml
rhFlt3L (manufactured by Peprotech, Inc., Cat. No. #300-19): 100 ng/ml
rhTPO (manufactured by Peprotech, Inc., Cat. No. #300-18): 20 ng/ml
rhVEGF (manufactured by Peprotech, Inc., Cat. No. #100-02): 50 ng/ml
rhIL-6 (manufactured by Peprotech, Inc., Cat. No. #200-06): 20 ng/ml
penicillin/streptomycin (manufactured by Gibco), Cat. No. 15140122: 100U/100 $\mu$g/ml
r: recombinant
h: Human origin

The PBMNC was seeded in a 10 cm dish (manufactured by Sumitomo Bakelite Co., Ltd.) so as to achieve 10 $\times 10^7$ cells/10 ml/dish and then cultured for 7 days at 37°C and 5% $CO_2$ in a humid environment. From this, a regenerative cell population (RAC) was prepared. In the following, unless otherwise specified, the culturing conditions were at 37°C and 5% $CO_2$ in a humid environment.

(2) Preparation of Extracellular Vesicles

After the culturing, the RAC was recovered and then centrifuged. The obtained pellet was suspended in an extracellular vesicle production medium, thereby preparing a cell suspension. The extracellular vesicle production medium was composed of X-vivo 15 Media (manufactured by Lonza, Cat. No.BE02-054Q) containing 5% (v/v) of exosome-removed fetal calf sera. The resulting cell suspension was seeded in a 10 cm dish so as to achieve $2.5 \times 10^5$ cells/ml/dish (250 k) or $5 \times 10^5$ cells/ml/dish (500 k), and then cultured for 48 hours.

After the culturing, the culture supernatant (conditioned medium) was recovered, and then the culture supernatant was centrifuged at $300 \times$ g for 10 minutes at 4°C to remove the cells. The resulting supernatant fraction was then centrifuged at $2000 \times$ g for 20 minutes at 12°C and then the supernatant fraction was recovered to remove apoptotic vesicles and microvesicles. The supernatant fraction was filtered through a 0.2-$\mu$m filter (manufactured by Millipore Merck, Cat. No. SLGS033SS) to remove extracellular vesicles having a particle size exceeding 200 nm. Then, the obtained filtrate was centrifuged at $174000 \times$ g and 4°C for 110 minutes to precipitate extracellular vesicles (exosomes) to recover. The recovered precipitate was suspended in the medium or buffer (up to $3 \times 50$ $\mu$l) for use in each experiment described below. In order to label the extracellular vesicles, after the 0.2-$\mu$m filter filtration, CM-Dil dye (C7000, manufactured by Thermo Fisher Scientific Inc.) was added to the filtrate so as to achieve 2 $\mu$mol/l. Then, the obtained reactant was centrifuged at $174000 \times$ g at 4°C for 110 minutes, and then the extracellular vesicles were washed with PBS and centrifuged under the same conditions. The same washing and centrifugation were performed again. The recovered precipitate was then suspended in the medium or buffer (up to $3 \times 50$ $\mu$l) for use in each experiment described below.

(3) Characteristics of Extracellular Vesicles

The extracellular vesicles prepared in Example 1(2) were examined for their mean diameter, amount of production per cell, expression of markers, and structure of lipid membranes and lumens. Specifically, the mean diameter, the amount of production per cell, and the expression of markers were measured by flow cytometry. As to the flow cytometer, DxFlex Flow Cytometry (manufactured by Beckman Coulter, Inc.) with three lasers (405 nm, 488 nm, and 638 nm) was used after changing its configuration as follows. The 405/10 VSSC filter was moved to V450 channel in WDM. Then, the configuration of the detector in the software (CytExpert software) was updated and assigned to VSSC channels in WDM. The flow path of the flow cytometer was washed with a washing solution (FlowClean) and then filtered through a 10-$\mu$m filter.

The flow cytometer (DxFlex FACS machine, manufactured by Beckman Coulter, Inc.) was calibrated using latex beads with different sizes (100 nm or 200 nm), and then the extracellular vesicles were measured. Further, with respect to the intensity of the obtained side scattered light (SSC), the particle size of each extracellular vesicle was calculated by comparing the intensity of the latex beads with the intensity of each extracellular vesicle. Then, the mean diameter (weighted average) was calculated based on the obtained particle size. In addition, the particle sizes of the extracellular vesicles were calculated, and the mean diameter (weighted average) was calculated in the same manner except that a nanoparticle tracking analyzer (NTA) (ZetaView®, manufactured by DKSH Holding Ltd.) was used instead of the flow cytometer. Then, it was verified that the mean diameter obtained by the flow cytometer and the mean diameter obtained by NTA were substantially the same.

The expression of the markers was measured using the flow cytometer after staining the extracellular vesicles with an anti CD9 antibody (PE labeling, manufactured by Biolegend Inc., Cat. No. #312106) or an anti CD63 antibody (PE labeling, manufactured by Biolegend Inc., Cat. No. #353004) and an anti Alix antibody (Alexa 594 labeling, manufactured by Biolegend Inc., Cat. No. #634504) and an anti Hsp-70 antibody (Alexa 488 labeling, manufactured by Biolegend Inc., Cat. No. #648003). As a control, the above procedure was conducted in the same manner except that the control antibody was used instead of the various antibodies.

As to the amount of production per cell, the amount of production of extracellular vesicles (the number of extracellular vesicles) per $2.5 \times 10^5$ cells was calculated based on the counts in the flow cytometer and the measured sample volume.

The structure of the lipid membrane and the lumen were observed using a scanning electron microscope (JEM-1400, manufactured by JEOL Ltd.).

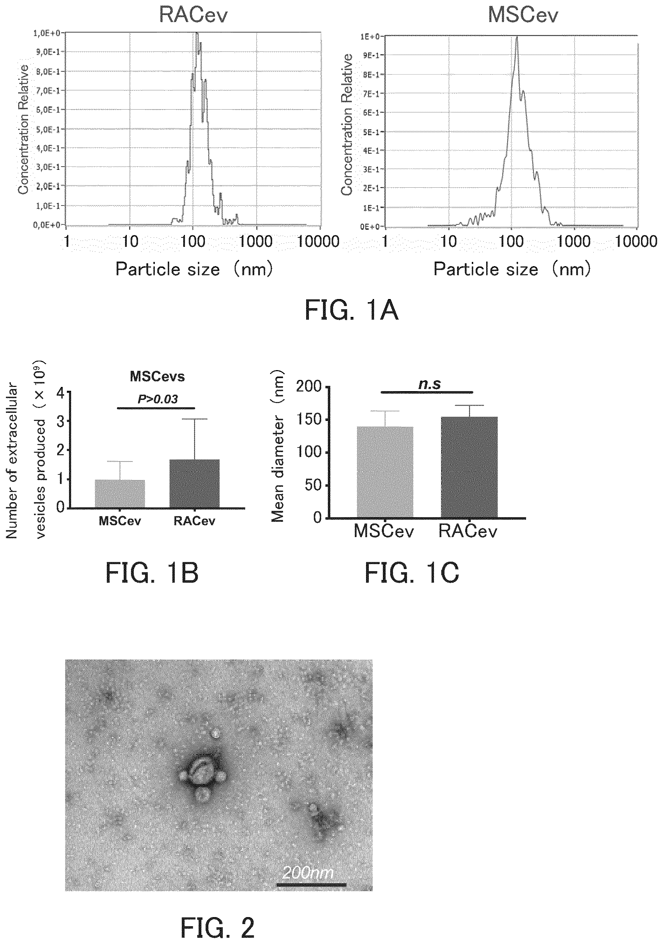

As a reference, the above procedure was conducted in the same manner except that human-derived mesenchymal stem cells (Cat. No.BE02-054Q, manufactured by MSC, Lonza) were used instead of the RAC. These results are shown in FIGs. 1A to 3B. The statistical processing in the graph was performed using a Mann-Whitney test.

FIGs. 1A to 1C are graphs showing the particle size, amount of production per cell, and mean diameter of extracellular vesicles. FIG. 1A shows the result of the particle size, FIG. 1B shows the result of the production amount, and FIG. 1C shows the result of the mean diameter. In FIG. 1A, the horizontal axis indicates the particle size (logarithm), and the vertical axis indicates the relative number. In FIG. 1B, the horizontal axis indicates the type of sample, and the vertical axis indicates the number of extracellular vesicles produced. In FIG. 1C, the horizontal axis indicates the type of sample, and the vertical axis indicates the mean diameter. As shown in FIGs. 1A and 1C, RAC-derived extracellular vesicles (RACev) were slightly larger than MSC-derived extracellular vesicles (MSCev), and the mean diameter of RACev was 145 nm and the mean diameter of MSCev was 130 nm. In addition, as shown in FIG. 1B, RAC produced significantly more extracellular vesicles per cell than MSC.

Next, FIG. 2 is an electron-microscopic image showing the structure of RAC-derived extracellular vesicles. As shown in FIG. 2, it was observed that RACev has a lipid bilayer membrane structure and a lumen, and the lumen is surrounded by the lipid bilayer membrane, and that RACev has a structure similar to that of an extracellular vesicle such as MSCev.

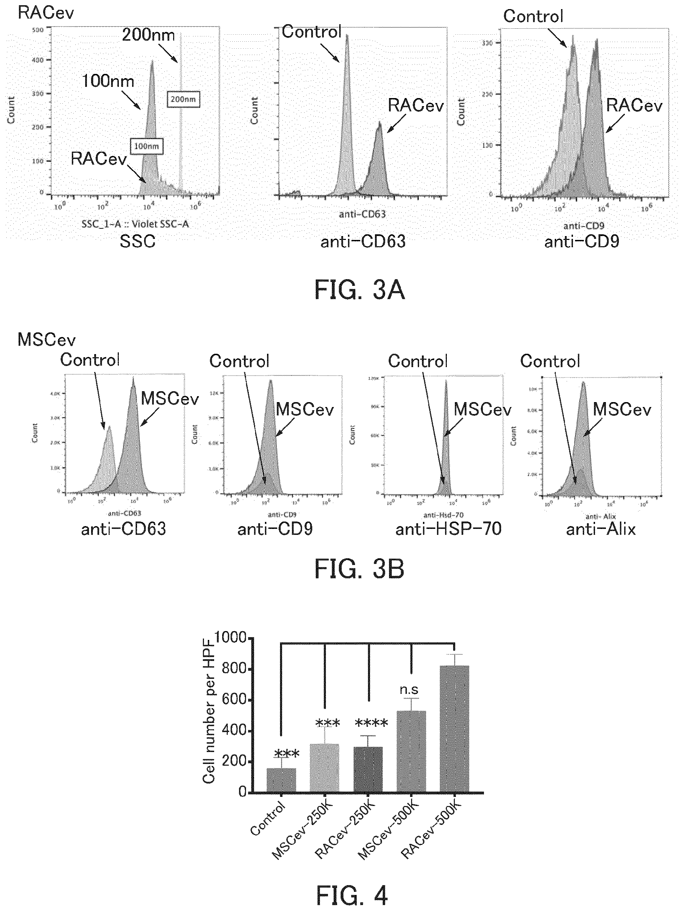

FIGs. 3A and 3B are histograms each showing the expression of a marker. FIG. 3A shows the result of RACev and FIG. 3B shows the result of MSCev. In each of FIGs. 3A and 3B, the horizontal axis indicates the intensity of SSC or the fluorescent intensity of each marker, and the vertical axis indicates the count. As shown in the left graph of FIG. 3A, it was found that the particle size of the extracellular vesicle can be measured by a flow cytometer by using latex beads having known particle sizes. In addition, as shown in the center graph and the right graph of FIG. 3A, the expressions of CD63 and CD9 were observed in RACev. Further, as shown in FIGs. 3A and FIG. 3B, the expression level of CD63 was higher in RACev than that of in MSCev. Although it is not shown in FIG. 3A, the expression of Alix and HSP70 was observed also in RACev. These results show that RACev expresses exosomal markers and is distinguishable from MSCev by the expression of CD9 and Alix.

These results show that RACev meets MISEV2018 (Thery C et.al., 2018, J.Extracell Vesicles 7:1535750, herein incorporated by reference), and therefore is an extracellular vesicle.

(4) Vascular Endothelial Cell Growth Promoting Activity

The vascular endothelial cell growth promoting activity of RACev was examined using human fetal-derived vascular endothelial cells (HUVEC, manufactured by Lonza, Cat. No.C2517A, Batch #. 18TL061650). Specifically, HUVEC cells were labeled by recovering HUVEC that had been subcultured 3 to 4 times, adding Cytopainter dye (manufactured by abcam, Cat. No. #ab176735) and incubating at 37°C for 10 to 30 minutes. After the labeling, centrifugation and washing were repeated twice. The obtained HUVEC cells were seeded in 3.5-cm dish so as to achieve $5 \times 10^4$ cells/2ml/dish. The medium for subclturing HUVEC cells was EGM-2 (manufactured by Lonza), and the medium for measuring the HUVEC cell growth activity was EBM-2 (manufactured by Lonza). Extracellular vesicles (RACev or MSCev) isolated from RAC or MSC of 250k or 500k were then added to the respective dishes. Then, the cells were cultured under the above-described culturing conditions for 2 days. After the culturing, the dishes were observed using a phase contrast microscope (6-well dish, manufactured by Falcon). In the obtained retardation image, the number of cells per field (HPF) was counted, and the proportion of the region occupied by HUVEC cells to the entire area was calculated as the growth area.

Further, after the culturing, HUVEC cells were recovered from the dishes, and the division of HUVEC cells was measured using a flow cytometer (FACS Verse, manufactured by BD Biosciences Co., Ltd.) with the label as an indicator. As a control, the above procedure was conducted in the same manner except that no RACev or MSCev were added.

In addition, RACev and MSCev added during culturing of HUVEC cells were analyzed for miRNA. Specifically, as to RACev and MSCev, miRNA was purified using a miRNA purification kit (miRNeasy mini kit and RNeasy MinElute Cleanup Kit, manufactured by QIAGEN). A library for sequencing was prepared using the obtained miRNA and a library construction kit (QIAseq™ miRNA Library Kit, QIAGEN, Cat. No. #331505). The quality-testing of the libraries was performed using Agilent Bioanalyze and DNA tips (High Sensitivity DNA chip, manufactured by Agilent Technologies, Inc.). The sequencing of the library was decoded as a 76 bp(bp) single-ended read using the next generation sequencer (NextSeq 500, manufactured by Illumina, Inc.). The expression levels of the respective miRNAs (miR-181b-3p, miR-150-5p, miR-302a-5p, and miR-92a-2-5p) were calculated from the obtained sequence data. Relative expression levels were calculated based on the expression levels of the respective miRNAs in HUVEC cells to which no extracellular vesicles were added.

These results are shown in FIGs. 4 to 6. As a control, the above procedure was conducted in the same manner except that no extracellular vesicles were added. As the statistical processing in the graph, a multiple comparison test of Tukey was performed after Two-way ANOVA test.

FIG. 4 is a graph showing the HUVEC cell growth. In FIG. 4, the horizontal axis indicates the type of sample, and the vertical axis indicates the number of cells per field of view (HPF). As shown in FIG. 4, RACev and MSCev promoted the vascular endothelial cell growth as compared to that of the control. Also, as shown in FIG. 4, RACev significantly promoted the vascular endothelial cell growth as compared to that of MSCev (MSCev-250K vs. RACev-250K: P > 0.001, MSCev-500K vs. RACev-500K: P > 0.0001).

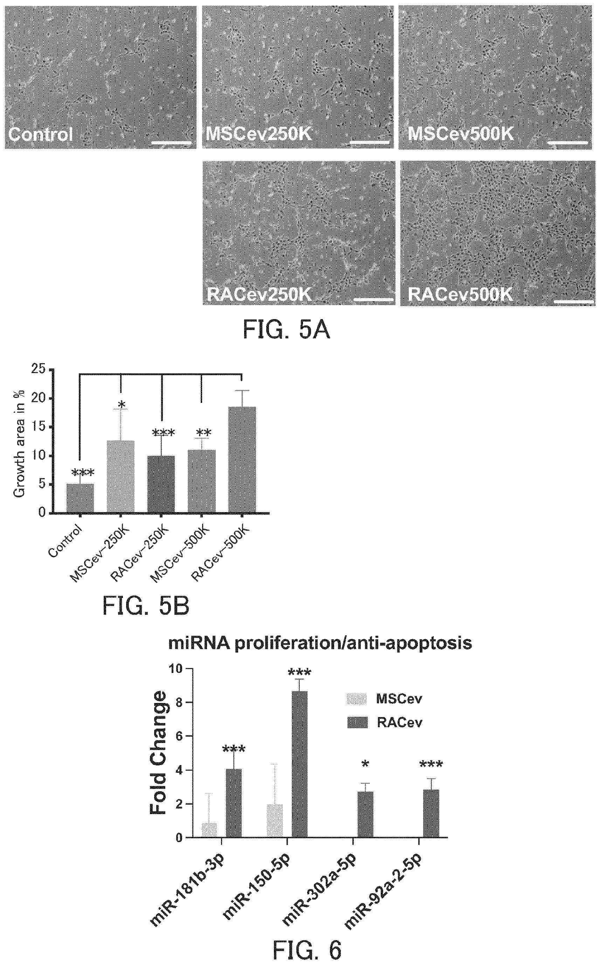

Next, FIGs. 5A and 5B are photographs and a graph showing the growth area of HUVEC cell. FIG. 5A shows phase difference images, and FIG. 5B shows a graph of a growth area. In FIG. 5B, the horizontal axis indicates the type of the sample, and the vertical axis indicates the growth area. As shown in FIGs. 5A and 5B, RACev and MSCev had an increased growth area of vascular endothelial cells as compared to that of the control. Also, as shown in FIGs. 5A and 5B, RACev significantly increased the growth area of vascular endothelial cells as compared to that of MSCev (MSCev-500K vs. RACev-500K: P > 0.0007). Incidentally, although it is not shown, during observation of the HUVEC cell growth over time using labeled RACev, it was observed that when the HUVEC cells incorporate RACev, the HUVEC cells proceeded to the S phase. It was also verified that the intravascular cell growth promoting activity by RACev was maintained up to the eighth generation, i.e., maintained during eight divisions.

Next, FIG. 6 is a graph showing the expression level of miRNA. In FIG. 6, the horizontal axis indicates the type of miRNA, and the vertical axis indicates the relative expression level. As shown in FIG. 6, RACev had significantly high expression levels of miRNA (miR-92a-2-5p) having anti-apoptotic activity, miRNAs (miR-181b-3p, miR-150-5p, and miR-302a-5p) having cell division promoting activity, and miRNA (miR-150-5p) having cell migration promoting activity as compared to that of MSCev. From these results, it was found that RACev exhibits the vascular endothelial cell growth promoting activity by transporting miRNA having anti-apoptotic activity and miRNA having cell division promoting activity.

(5) Improvement of Ischemic Heart Disease

In ischemic heart disease, angiogenesis, caused by vascular endothelial cell growth, improves prognosis. Therefore, it was examined that RACev contributes to the improvement of ischemic heart diseases using rat myocardial infarction models. Specifically, Lewis rats (male, 6-10 weeks old, bodyweight: 150 to 250 g, purchased from Charles River Laboratories Japan) were intubated and then anesthetized by causing them to inhale 3 to 4% sevoflurane (manufactured by Maruishi Pharmaceutical Co., Ltd.) with 15 ml/kg for 65 to 70 minutes. After left thoracotomy, the left anterior descending artery (LAD) was left in ischemia for 30 minutes and then reperfused, thereby inducing ischemia-reperfusion injury. After the onset, the thorax was immediately closed. The extracellular vesicles (RACev or MSCev) isolated from RAC or MSC of 500 k were then diluted with PBS and the resulting dilution was administered from the tail vein at predetermined times (30 minutes, 1 day, and 2 days) after inducing the ischemia-reperfusion injury.

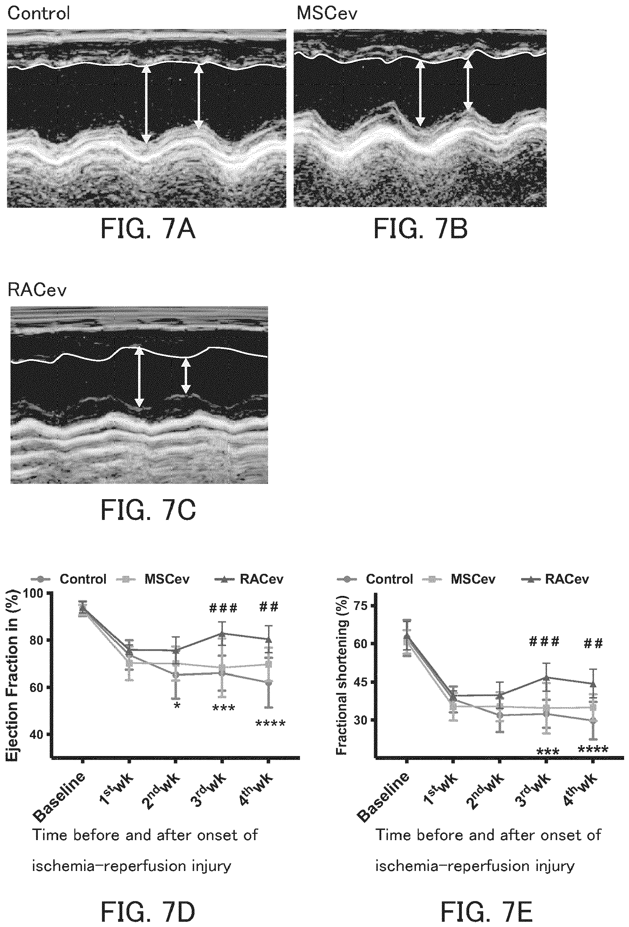

The severity of ischemic heart disease was evaluated by echocardiography. Specifically, the severity grades of the left ventricular end-diastolic diameter (LVDD), left ventricular end-systolic diameter (LVDS), and mitral regurgitation (MR), the height of E-wave and A-wave, and the size of left atrial and ascending aortic were measured using an echocardiographic apparatus (made by ALOCA) equipped with a 3.5 MHz probe. The measurement was performed first before onset of the ischemia-reperfusion injury (baseline) and then once a week until 4 weeks after onset of the injury. The mitral regurgitation severity grade was evaluated according to the following criteria. The left ventricular fractional shortening (%FS) was calculated by Teichholz method (Salybekov et. al., PLoSONE, (2018), 13(11):e0203244; herein incorporated by reference). Specifically, the left ventricular fractional shortening was calculated by the following equation (1). These measured values were also used to calculate ejection fraction and left ventricular end-systolic volume (LVESV). As a control, calculation was carried out in the same manner except that no RACev or MSCev was administered. These results are shown in FIGs. 7 to 8. As the statistical processing in the graph, a multiple comparison test of Tukey was performed after Two-way ANOVA test.

$$\%FS = (LVDD - LVDS) / LVDD \times 100 \quad (1)$$

(Evaluation of Severity)

Score 3 (severe): reflux reaching left atrial wall
Score 2 (moderate): reflux reaching 2/3 of left atrium
Score 1 (mild/slight): reflux reaching 1/3 of left atrium

FIGs. 7A to 7E are photographs each showing the echocardiogram and graphs showing the ejection fraction and the left ventricular fractional shortening. FIG. 7A shows the result of control, FIG. 7B shows the result of MSCev, FIG. 7C shows the result of RACev, FIG. 7D shows the result of ejection fraction, and FIG. 7E shows the result of left ventricular internal diameter shortening fraction. In each of FIGs. 7A to 7C, the long arrow indicates an example of LVDD, and the short arrow indicates an example of LVDS. In FIG. 7D, the horizontal axis indicates the time before and after onset of the ischemia-reperfusion injury, and the vertical axis indicates the ejection fraction. In FIG. 7E, the horizontal axis indicates the time before and after onset of the ischemia-reperfusion injury, and the vertical axis indicates the left ventricular fractional shortening. As shown in FIGs. 7A to 7E, RACev had significantly improved ejection fraction and significantly improved left ventricular fractional shortening as compared to that of the control and MSCev.

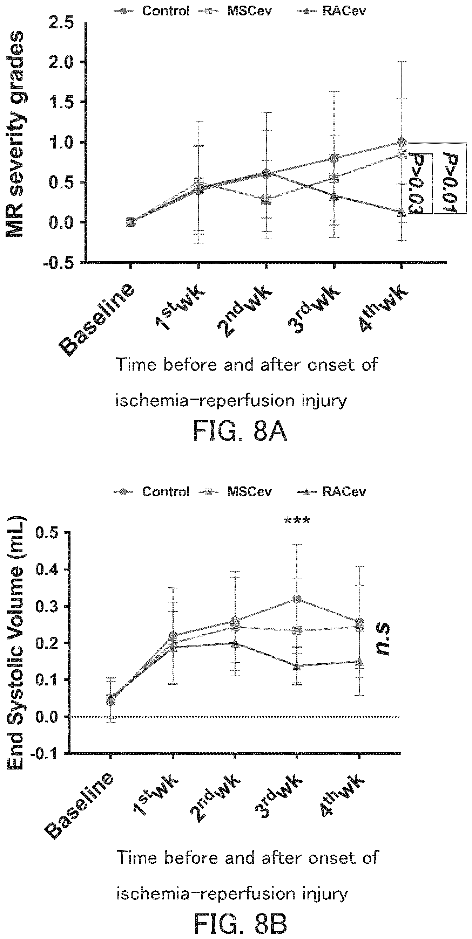

FIGs. 8A and 8B are graphs showing the mitral regurgitation severity grade and left ventricular end-systolic volume. FIG. 8A shows the result of the mitral regurgitation severity grade, and FIG. 8B shows the result of left ventricular end-systolic volume. In FIG. 8A, the horizontal axis indicates the time before and after onset of the ischemia-reperfusion injury, and the vertical axis indicates the mitral regurgitation severity grade. In FIG. 8B, the horizontal axis indicates the time before and after onset of the ischemia-reperfusion injury, and the vertical axis indicates the left ventricular end-systolic volume. As shown in FIG. 8A, RACev improved the mitral regurgitation severity grade as compared to that of the control and MSCev and returned to normal level 4 weeks after the ischemia-reperfusion injury. Also, as shown in FIG. 8B, RACev improved left ventricular end-systolic volume as compared to that of the control and MSCev, which is presumed to be because RACev improved the severity of mitral regurgitation.

These results showed that RACev can improve ischemia-reperfusion injury in the heart.

(6) Suppression of Fibrosis in Ischemic Heart Disease

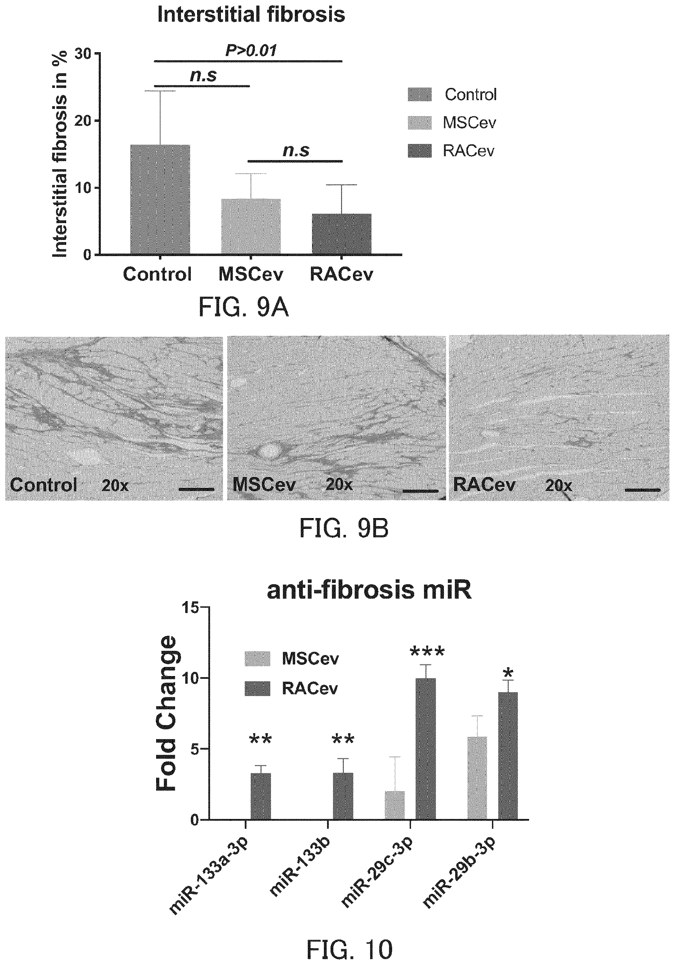

Ischemic heart disease due to the ischemia-reperfusion injury causes fibrosis. Hence, it was examined whether the fibrosis was suppressed by the administration of RACev. Hearts were collected from the rats 4 weeks after the onset of ischemia-reperfusion injury in Example 1(5). The hearts were perfused with 1000 U/ml heparin-containing PBS and immobilized overnight (about 8 hours) with 4% paraformaldehyde (manufactured by FUJIFILM Corporation, Cat. No. #163-20145). After the immobilization, the hearts were embedded in paraffin and paraffin-embedded sections having a 3 to 4 $\mu$m thickness were prepared. For the sections, after staining with picrosirius red, the proportion (%) of interstitial fibrosis in a single field of view was determined (see Salybekov AA et.al., 2019, PLoS One 14: e0205477; herein incorporated by reference). As a control, the above procedure was conducted in the same manner except that no RACev or MSCev was administered. As to RACev and MSCev added at the time of culturing HUVEC cells, the relative expression levels of the respective miRNAs (miR-133a-3p, miR-133b, miR-29c-3p, and miR-29b-3p) were calculated in the same manner as in Example 1(4). These results are shown in FIGs. 9A to FIG. 10. Dunn's multiple comparison test was performed as the statistical processing in the graph.

FIGs. 9A and 9B are a graph and photographs showing the degree of fibrosis. FIG. 9A shows the size of interstitial fibrosis, and FIG. 9B shows representative examples of the respective samples. In each of the photographs of FIG. 9B, a dark gray area indicates an area with fibrosis. In FIG. 9A, the horizontal axis indicates the type of sample, and the vertical axis indicates the size of interstitial fibrosis. As shown in FIGs. 9A and 9B, RACev and MSCev significantly suppressed the degree of fibrosis as compared to that of the control.

Next, FIG. 10 is a graph showing the expression level of miRNA. In FIG. 10, the horizontal axis indicates the type of miRNA, and the vertical axis indicates the relative expression level. As shown in FIG. 10, RACev had significantly high expression levels of miRNAs having anti-fibrotic activity as compared to those of MSCev. These results show that RACev exhibits the fibrosis suppressing activity by transporting miRNA having fibrosis suppressing activity.

(7) Angiogenesis Promoting Activity of Extracellular Vesicles

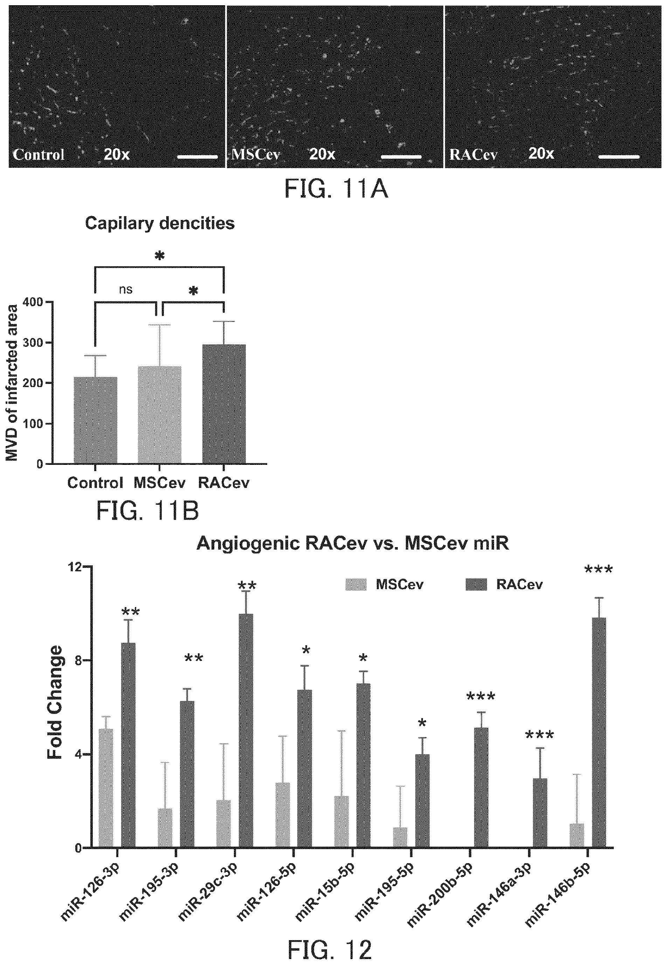

It was examined whether the administration of RACev promotes angiogenesis. The paraffin-embedded sections prepared in Example 1 (6) were blocked with a complex of avidin and streptavidin, then stained with 100-fold diluted Isolectin B4-FITC (manufactured by Invitrogen Corporation), and 200-fold diluted $\alpha$SMA-Cy3 (Sigma Chemical Co.), thereby staining the microvessels and arterioles in the infarcted myocardial tissue. The obtained sections were observed using a fluorescence microscopy (FSX100, HPF 20×, manufactured by OLYMPUS CORPORATION) or a confocal microscopy (LSM880, manufactured by Carl Zeiss AG). The density of the blood vessel was calculated from the obtained microscopic image. As a control, the above procedure was conducted in the same manner except that no RACev or MSCev was administered. As to RACev and MSCev added at the time of culturing HUVEC cells, the relative expression levels of the respective miRNAs (miR-126-3p, m miR-195-3p, miR-29c-3p, miR-126-5p, miR-15b-5p, miR-195-5p, miR-200b-5p, miR-146a-3p, and miR-146-5p) were calculated in the same manner as in Example 1(4). These results are shown in FIGs. 11A to FIG. 12. Dunn's multiple comparison test was performed as the statistical processing in the graph.

FIGs. 11A and 11B are photographs each showing the blood vessel in infarcted myocardial tissue and a graph. FIG. 11A shows the blood vessel in infarcted myocardial tissue, and FIG. 11B shows the density of blood vessels. In FIG. 11B, the horizontal axis indicates the type of the sample, and the vertical axis indicates the microvessel density in the infarcted myocardial tissue. As shown in FIGs. 11A and 11B, RACev had significantly increased vascular density as compared to that of the control and MSCev.

Next, FIG. 12 is a graph showing the expression level of miRNA. In FIG. 12, the horizontal axis indicates the type of miRNA, and the vertical axis indicates the relative expression level. As shown in FIG. 12, RACev had significantly high expression levels of miRNAs having angiogenesis promoting activity as compared to those of MSCev. These results show that RACev exhibits the angiogenesis promoting activity by transporting miRNA having angiogenesis promoting activity.

(8) Tropism of Extracellular Vesicles to Inflammatory Sites

Since RACev contributes to improvement of ischemia-reperfusion injury, RACev is presumed to be accumulated *in vivo* at the inflammation site caused by ischemia-reperfusion. Hence, the time for incorporating RACev into HUVEC cells was examined. Specifically, the labeled RACev prepared in Example 1(2) was added to a dish seeded with HUVEC cells in advance, and the label was detected over time to detect RACev. As a result, it was found that the RACev is incorporated into HUVEC cells within 3 to 4 hours after the addition.

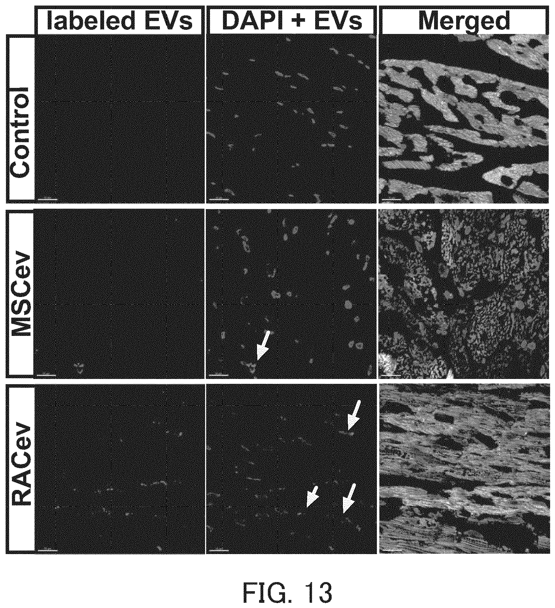

As in Example 1(5), ischemia-reperfusion injury was induced, and after the onset of ischemia-reperfusion injury, the thorax was immediately closed. The extracellular vesicles (RACev or MSCev) isolated from RAC or MSC of 500 k were then diluted with PBS and the resulting dilution was administered from the tail vein immediately after inducing the ischemia-reperfusion injury. The hearts were collected from the rats 3.5 hours after the administration. Whether RACev is accumulated in the infarcted myocardial tissue of the heart was measured using a fluorescent imaging system (IVIS Lumina III, manufactured by PerkinElmer Inc.). The excitation wavelength was 520 nm, and the emission wavelength was 570 nm. From the obtained measured values, luminance values per unit area were calculated. In addition, a frozen section having a thickness of 6 $\mu$m was prepared from the collected heart, and the obtained frozen section was observed using a confocal microscope (LSM880, manufactured by Carl Zeiss AG) after nuclear staining. As a control, the above procedure was conducted in the same manner except that no RACev or MSCev was administered. These results are shown in FIGs. 13 to 14B. The statistical processing in the graph was performed using the Mann-Whitney test.

FIG. 13 shows photographs each showing fluorescent image of infarcted myocardial tissue. In FIG. 13, the upper row indicates the results of Control, the middle row indicates the results of MSCev, and the lower row indicates the results of RACev. Also, in FIG. 13, the left column shows the detection results of labeled extracellular vesicles (RACev or MSCev), the middle column shows the detection results of labeled extracellular vesicles (RACev or MSCev) and nuclei, and the right column shows these merging results. As shown in FIG. 13, RACev and MSCev were found to be accumulated in infarcted myocardial tissue. RACev is accumulated more than MSCev, and, as indicated by arrows in the photographs, it was observed that RACev was incorporated in the nuclei of cardiomyocytes.

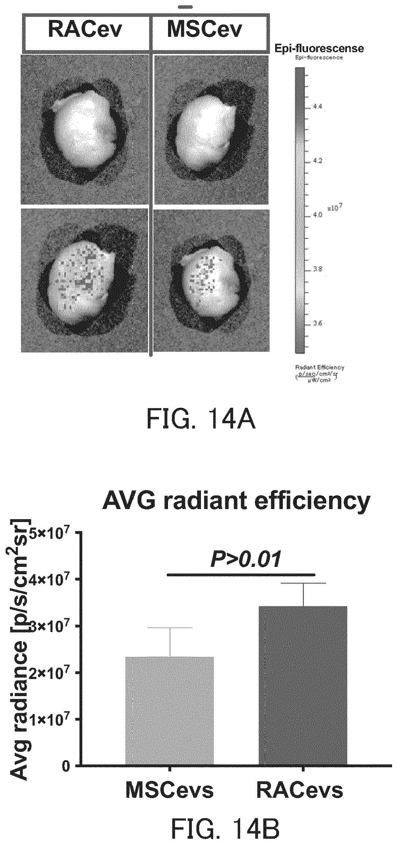

FIGs. 14A and 14B are photographs each showing the luminescence intensity of infarcted myocardial tissue and a graph. FIG. 14A shows photographs, each showing the luminescence intensity of infarcted myocardial tissue, and FIG. 14B is a graph showing the luminescence intensity per unit area. As shown in FIGs. 14A and 14B, RACev was observed to be accumulated comparatively more than MSCev in infarcted myocardial tissue of extracellular vesicles. From the above, it was verified that RACev is excellent in accumulation in inflammatory tissue.

(9) Suppression of Transplant Rejection

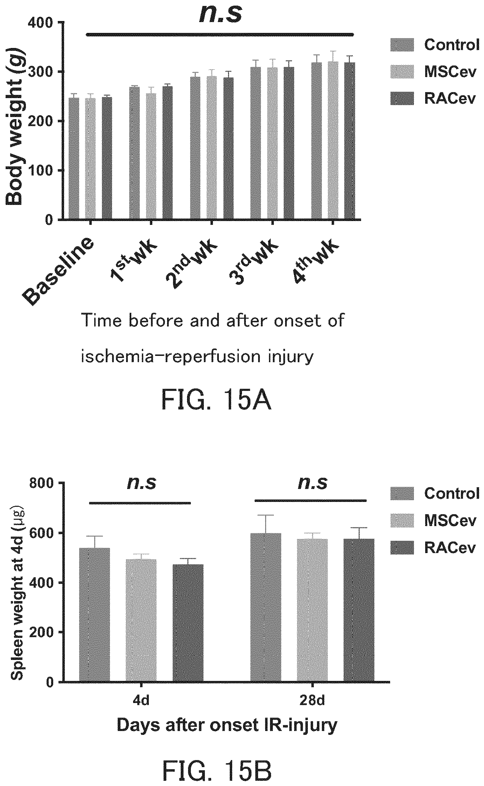

It was examined that there was no transplant rejection after the administration of RACev. As in Example 1(5), ischemia-reperfusion injury was induced. After the onset of ischemia-reperfusion injury, the thorax was immediately closed. The extracellular vesicles (RACev or MSCev) isolated from RAC or MSC of 500 k were then diluted with PBS and the resulting dilution was administered from the tail vein at predetermined times (30 minutes, 1 day, and 2 days) after inducing the ischemia-reperfusion injury. The first body weight measurement was then performed before the onset of the ischemia-reperfusion injury (baseline), and then the body weight was measured once a week until 4 weeks after the onset of the injury. Spleens were collected and weighed 4 days and 4 weeks after the onset. In addition, peripheral blood and spleen were collected 4 weeks after the onset. The proportion of CD3 positive cells, CD4 positive cells, CD8 positive cells, regulatory T cells, and CD11b/c positive cells in the peripheral blood was measured using a flow cytometer. In addition, the proportion of CD3 positive cells, CD4 positive cells, CD8 positive cells, TNK, iNK, antigen-presenting cells, and regulatory T cells, which are markers of transplant rejection in the spleen, in the spleen was measured using a flow cytometer. As a control, the above procedure was conducted in the same manner except that no RACev or MSCev was administered. As to RACev and MSCev administered, the relative expression level of the respective miRNA (miR-142-3p) was calculated in the same manner as in Example 1(4). These results are shown in FIGs. 15A to FIG. 18. Dunn's multiple comparison test was performed as the statistical processing in the graph.

FIGs. 15A and 15B are graphs showing the body weight and the spleen weight of rat. FIG. 15A shows the results of body weight of rats and FIG. 15B shows the results of spleen weight of rats. In FIG. 15A, the horizontal axis indicates the measurement time based on onset of the ischemia-reperfusion, and the vertical axis indicates the body weight of rats. In FIG. 15B, the horizontal axis indicates the measurement time based on onset of the ischemia-reperfusion (IR-injury), and the vertical axis indicates the spleen weight of rats. As shown in FIGs. 15A and 15B, there were no differences among RACev, MSCev, and controls, and no transplant rejection was observed.

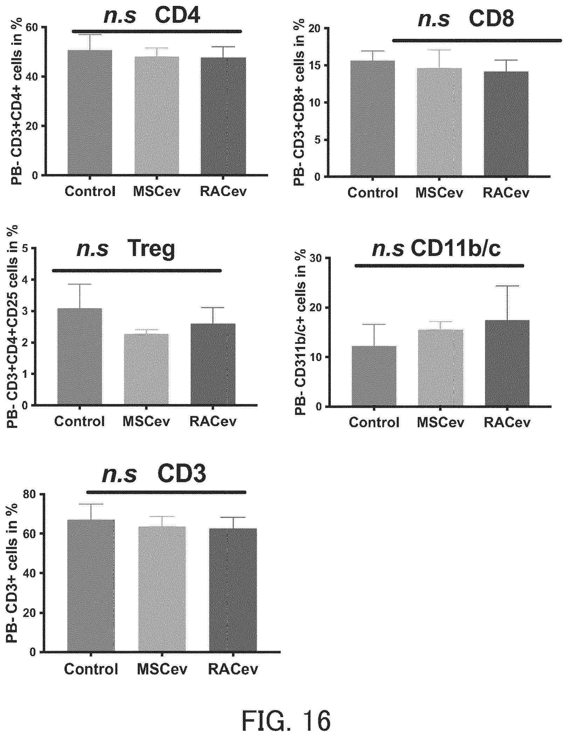

FIG. 16 shows graphs showing the proportion of CD3 positive cell, CD4 positive cell, CD8 positive cell, regulatory T cell, and CD11b/c positive cell. In FIG. 16, the horizontal axis indicates the type of sample, and the vertical axis indicates the proportion of each cell. As shown in FIG. 16, there were no differences among RACev, MSCev, and controls, and no transplant rejection was observed.

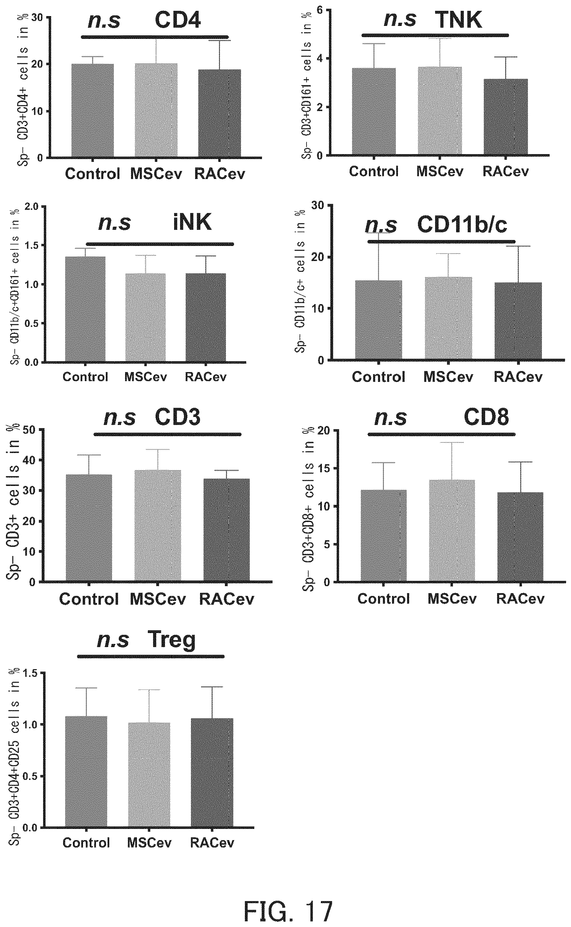

FIG. 17 shows graphs showing the proportion of CD3 positive cell, CD4 positive cell, CD8 positive cell, TNK, iNK, regulatory T cell, and CD11b/c positive cell. In FIG. 17, the horizontal axis indicates the type of sample, and the vertical axis indicates the proportion of each cell. As shown in FIG. 17, there were no differences among RACev, MSCev, and controls, and no transplant rejection was observed.

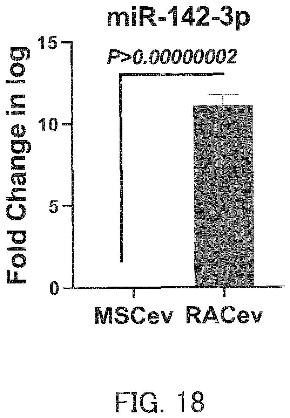

FIG. 18 is a graph showing the expression level of miRNA. In FIG. 18, the horizontal axis indicates the type of sample, and the vertical axis indicates the relative expression level. As shown in FIG. 18, RACev had a significantly high expression level of miRNA (miR-142-3p) having immunosuppressive activity (anti-inflammatory properties) as compared to that of MSCev. It is known that miR-142-3p is contained in exosomes released by regulatory T cells (Treg). From these results, it was suggested that RACev suppressed transplant rejection by transporting miRNA having immunosuppressive activity.

From the above, it was found that extracellular vesicles of a regenerative cell population, that is, the extracellular vesicles of the present invention have vascular endothelial cell growth activity, angiogenesis promoting activity, and fibrosis suppressing activity, with reduced transplant rejection.

Example 2

It was examined that a regenerative cell population can be induced by culturing peripheral blood mononuclear cells or umbilical cord blood mononuclear cells with various factors.

(1) Preparation of Regenerative Cell Population

Peripheral blood mononuclear cells were prepared in the same manner as in Example 1(1). Umbilical cord blood mononuclear cells were prepared in the same manner except that umbilical cord blood was used instead of the peripheral blood.

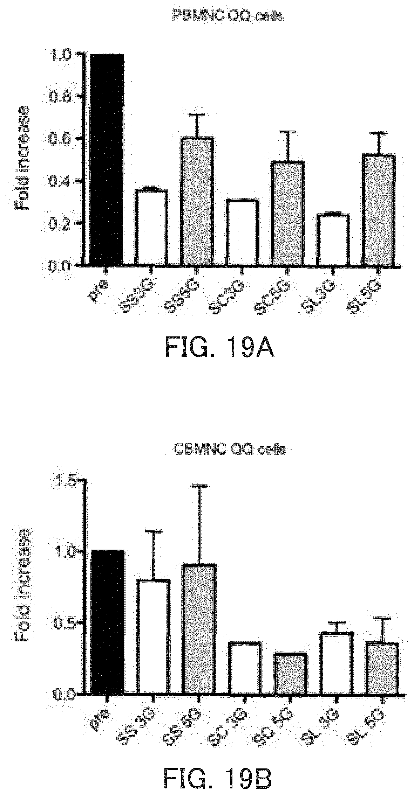

Next, as to the peripheral blood mononuclear cells and umbilical cord blood mononuclear cells, a regenerative cell population (RAC) was prepared in the same manner as in Example 1(1), except that the following Conditions 1 to 6 were used as the serum-free medium and the factors. The combination of the peripheral blood mononuclear cells and the following Condition 6 corresponds to the culturing condition of Example 1(1). After the culturing, the number of cells of the obtained regenerative cell population was counted. Then, the relative ratio of the number of cells was calculated based on the number of seeded cells. As a control, the peripheral blood mononuclear cells and umbilical cord blood mononuclear cells were used. These results are shown in FIGs. 19A and 19B.

(Type of QQ Medium and Concentration of Various Factors)

• Condition 1: SS3G

    Serum-free medium: StemSpan SFEM (manufactured by STEMCELL Technologies)
    Factors:

        rhSCF: 100 ng/ml
        rhFlt3L: 100 ng/ml
        rhTPO: 20 ng/ml

• Condition 2: SS5G

Serum-free medium: StemSpan SFEM
Factors:

rhSCF: 100 ng/ml
rhFlt3L: 100 ng/ml
rhTPO: 20 ng/ml
rhVEGF: 50 ng/ml
rhIL-6: 20 ng/ml

• Condition 3: SC3G

Serum-free medium: StemCell
Factors:

rhSCF: 100 ng/ml
rhFlt3L: 100 ng/ml
rhTPO: 20 ng/ml

• Condition 4: SC5G

Serum-free medium: StemCell
Factors:

rhSCF: 100 ng/ml
rhFlt3L: 100 ng/ml
rhTPO: 20 ng/ml
rhVEGF: 50 ng/ml
rhIL-6: 20 ng/ml

• Condition 5: SC3G

Serum-free medium: Stemline (manufactured by Sigma-Aldrich, Inc.)
Factors:

rhSCF: 100 ng/ml
rhFlt3L: 100 ng/ml
rhTPO: 20 ng/ml

• Condition 6: SL5G

Serum-free medium: Stemline

Factors:

rhSCF: 100 ng/ml
rhFlt3L: 100 ng/ml
rhTPO: 20 ng/ml
rhVEGF: 50 ng/ml
rhIL-6: 20 ng/ml

FIGs. 19A and 19B are graphs each showing the number of cells. FIG. 19A shows the results with peripheral blood mononuclear cells, and FIG. 19B shows the results with umbilical cord blood mononuclear cells. In each of FIGs. 19A and FIG. 19B, the horizontal axis indicates the condition, and the vertical axis indicates the relative ratio of the number of cells. As shown in FIG. 19A, using the peripheral blood mononuclear cells decreased the number of cells under all conditions. Using five factors increased the number of cells as compared to using three factors with any medium. As shown in FIG. 19B, using the umbilical cord blood mononuclear cells decreased the number of cells under all conditions. Using StemSpan SFEM as the serum-free medium increased the number of cells as compared to that of using other media.

(2) EPC Colonization Assays

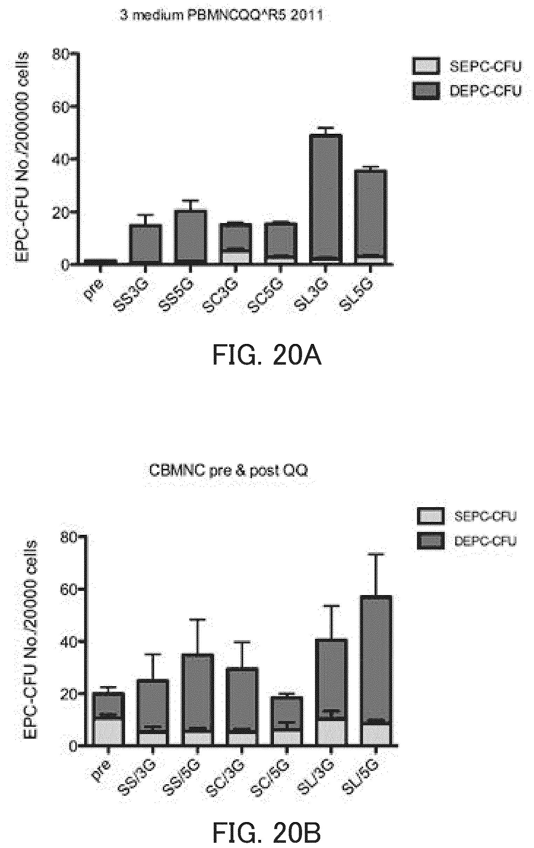

To examine the angiogenic potential of regenerative cell population, adhesive EPC colonies were quantified by EPC colony-forming assays (EPC-CFA). EPC-CFA was carried out according to the procedure described in Masuda H. et al., Circulation research, 109: 20-37 (2011), which is herein incorporated by reference. Specifically, the cells were cultured for 16 to 18 days in a semi-solid medium using 35 mm PRIMARIA™ dish (manufactured by BD Falcon). The composition of the semi-solid medium is shown in Table 1 below. After the culturing, using a phase contrast optical microscope (Eclipse TE300, manufactured by Nikon Corporation), the number of adhesive colonies per dish was measured with a gridded scoring dish (manufactured by STEMCELL Technologies). Undifferentiated EPC colonies (SEPC-CFU (primitive EPC colony forming unit) and differentiated EPC colonies (DEPC-CFU (definitive EPC colony forming unit) were counted separately. As a control (pre), the above procedure was conducted in the same manner except that the peripheral blood mononuclear cells or the umbilical cord blood mononuclear cells were used instead of the regenerative cell population. These results are shown in FIGs. 20A and 20B.

[Table 1]

|  | Manufacturer and Cat. No. | Final Concentration |
|---|---|---|
| METHOCULT™ SF$^{BIT}$ H4236 | Stem Cell Tec, #04236 |  |
| rh SCF | Peprotec, #300-07 | 100 ng/ml × 1.5 diluted |
| rh VEGF | Peprotec, #100-20 | 50 ng/ml × 1.5 diluted |
| rh basic FGF | Peprotec, #100-18B | 50 ng/ml × 1.5 diluted |
| rh EGF | Peprotec, #100-15 | 50 ng/ml × 1.5 diluted |
| rh IGF-1 | Peprotec, #100-11 | 50 ng/ml × 1.5 diluted |
| rh IL-3 | Peprotec, #200-03 | 20 ng/ml × 1.5 diluted |
| Heparin | Shimizu Pharmaceutical Co. | 2 IU/ml × 1.5 diluted |
| FBS | JRH Bioscience | 30% |

FIGs. 20A and 20B are graphs each showing the result of EPC-CFA assay. FIG. 20A shows the results with peripheral blood mononuclear cells, and FIG. 20B shows the results with umbilical cord blood mononuclear cells. In each of FIGs. 20A and 20B, the horizontal axis indicates the above conditions, and the vertical axis indicates the number of colonies of EPC cells per 20000 seeded cells. As shown in FIG. 20A, by culturing under the Conditions 1 to 6, the peripheral blood mononuclear cells hardly form SEPC-CFU and DEPC-CFU. In contrast, the peripheral blood mononuclear cell-derived cells improved the ability to form SEPC-CFU and DEPC-CFU. Since the ability to form SEPC-CFU and DEPC-CFU was improved under Conditions 1 to 5 as under Condition 6, it was suggested that the cell group induced by culturing the peripheral blood mononuclear cells under Conditions 1 to 5 was able to use as a regenerative cell population.
Next, as shown in FIG. 20B, in the umbilical cord blood mononuclear cells, SEPC-CFU and DEPC-CFU were formed to the same extent. In contrast, the peripheral blood mononuclear cell-derived cells improved the ability to form SEPC-CFU and DEPC-CFU by culturing under the Conditions 1 to 6. Since the ability to form SEPC-CFU and DEPC-CFU is improved also under Conditions 1 to 5 as under Condition 6, it was suggested that the cell group induced by culturing the umbilical cord blood mononuclear cells under Conditions 1 to 6 was able to use as a regenerative cell population.
From the above, it was verified that a regenerative cell population including EPC colony cells can be induced by culturing the peripheral blood mononuclear cells or the umbilical cord blood mononuclear cells with various factors.

Example 3

It was examined that the umbilical cord-derived extracellular vesicle of the present invention contains miRNA having angiogenesis promoting activity, miRNA having anti-apoptotic activity, miRNA having anti-fibrotic activity, miRNA having anti-fibrotic activity, and miRNA having cell growth promoting activity.

(1) Preparation of Umbilical Cord-Derived Regenerative Cell Population

An umbilical cord-derived regenerative cell population was prepared by inducing from human umbilical cord blood-derived mononuclear cells or human umbilical cord blood-derived CD34 positive progenitor cells. First, human umbilical cord blood-derived mononuclear cells (hereinafter, referred to as "CBMCs", manufactured by TAKARA BIO INC.) and human umbilical cord blood-derived CD34 positive endothelial progenitor cells (hereinafter, referred to as "CD34 cells", manufactured by TAKARA BIO INC.) were cultured using a QQ medium. The culturing with the QQ medium was performed using the method described in Example 1(1).

The CBMCs were seeded in a 10 cm dish (manufactured by Sumitomo Bakelite Co., Ltd.) so as to achieve $10 \times 10^7$ cells/10 ml/dish and then cultured for 7 days at 37°C and 5% $CO_2$ in a humid environment after seeding. The CD34 cells were seeded in a 10 cm dish (manufactured by Sumitomo Bakelite Co., Ltd.) so as to achieve $10 \times 10^5$ cell/10 ml/dish and then cultured for 7 days at 37°C and 5% $CO_2$ in a humid environment. From this, a regenerative cell population (RAC) was prepared.

(2) Preparation of Umbilical Cord-Derived Extracellular Vesicle

After the culturing, CBMCs or CD34 cells were recovered and suspended in an extracellular vesicle production medium, thereby preparing a cell suspension. The extracellular vesicle production medium was composed of X-vivo 15 Media (manufactured by Lonza, Cat. No.BE02-054Q) containing 5% (v/v) of exosome-removed fetal calf sera. After the preparation, the cells were incubated for 48 hours.

After the culturing, the culture supernatant (conditioned medium) was recovered, and then the culture supernatant was centrifuged at $300 \times g$ for 10 minutes at 4°C to remove the cells. The resulting supernatant fraction was then centrifuged at $2000 \times g$ for 20 minutes at 12°C and then the supernatant fraction was recovered to remove apoptotic vesicles and microvesicles. The supernatant fraction was filtered through a 0.2-$\mu$m filter (manufactured by Millipore Merck, Cat. No. SLGS033SS) to remove extracellular vesicles having a particle size exceeding 200 nm. Then, the obtained filtrate was centrifuged at $174000 \times g$ for 110 minutes at 4°C to precipitate and recover extracellular vesicles (exosomes).

(3) miRNA of Extracellular Vesicles of Umbilical Cord Blood-Derived Mononuclear Cells

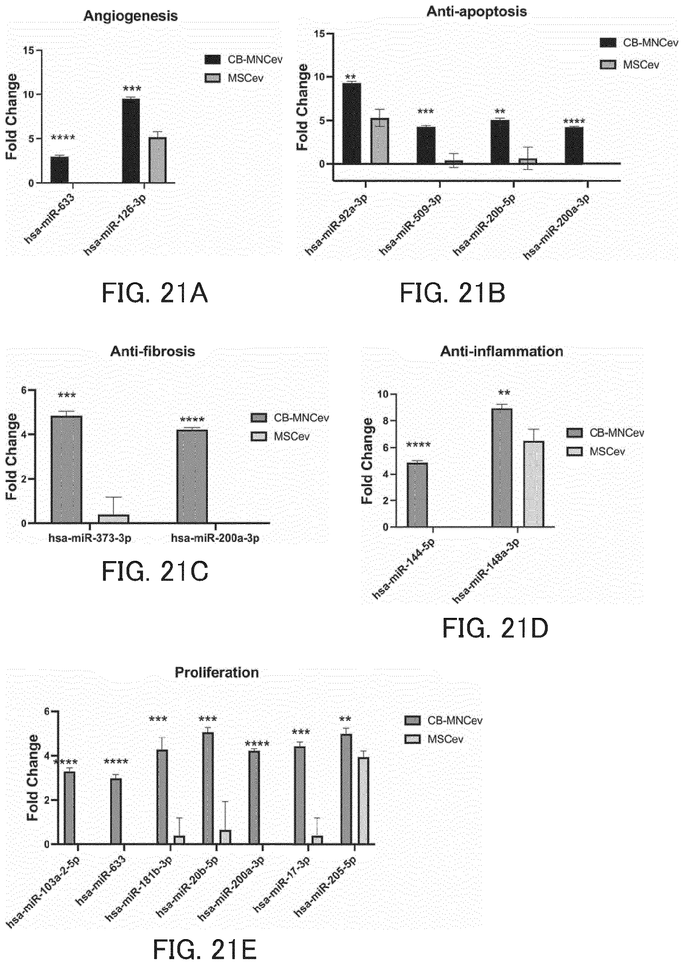

Extracellular vesicles (CB-MNCev) of umbilical cord blood-derived mononuclear cells and MSC-derived extracellular vesicles (MSCev) were analyzed for miRNA. Specifically, the analysis was carried out in the same manner as in Example 1(4). Relative expression levels of the respective miRNAs (miR-17-3p, miR-20b-5p, miR-92a-3p, miR-103a-2-5p, miR-126b-3p, miR-144-5p, miR-148a-3p, miR-181b-3p, miR-200a-3p, miR-205-5p, miR-373-3p, miR-509-3p, and miR-633) were calculated from the obtained sequence data. These results are shown in FIGs. 21A to 21E. As the statistical processing in the graph, a multiple comparison test of Tukey was performed after Two-way ANOVA test.

FIGs. 21A to 21E are graphs each showing the expression level of miRNA. FIG. 21A shows the expression level of miRNA having angiogenesis promoting activity, FIG. 21B shows the expression level of miRNA having anti-apoptotic activity, FIG. 21C shows the expression level of miRNA having anti-fibrotic activity, FIG. 21D shows the expression level of miRNA having anti-inflammatory activity, and FIG. 21E shows the expression level of miRNA having cell growth promoting activity. In each of FIGs. 21A to 21E, the horizontal axis indicates the type of RNA, and the vertical axis indicates the relative expression level. As shown in FIGs. 21A to 21E, CB-MNCev had significantly high expression levels of miRNAs (miR-633 and miR-126-3p) having angiogenesis promoting activity, miRNAs (miR-92a-3p, miR-509-3p, miR-20b-5p, and miR-200a-3p) having anti-apoptotic activity, miRNAs (miR-373-3p and miR-200a-3p) having anti-fibrotic activity, miRNAs (miR-144-5p and miR-148a-3p) having anti-inflammatory activity, and miRNAs (miR-103a-2-5p, miR-633, miR-181b-3p, miR-20b-5p, miR-200a-3p, miR-17-3p, and miR-205-5p) having cell growth promoting activity as compared to those of MSCev. From these results, it was found that CB-MNCev contains miRNA having angiogenesis promoting activity, miRNA having anti-apoptotic activity, miRNA having anti-fibrotic activity, and miRNA having cell growth promoting activity.

(4) miRNA of Extracellular Vesicles of Umbilical Cord Blood-Derived CD34 Positive Endothelial Progenitor Cells

Next, extracellular vesicles (CB-CD34ev) of umbilical cord blood-derived CD34 positive endothelial progenitor cells and MSC-derived extracellular vesicles (MSCev) were analyzed for miRNA. Specifically, the analysis was carried out in the same manner as in Example 1(4). Relative expression levels of the respective miRNAs (miR-10a-3p, miR-17-3p, miR-20b-5p, miR-21-5p, miR-24-2-5p, miR-29a-5p, miR-29c-3p, miR-30b-5p, miR-30c-5p, miR-30e-3p, miR-30e-5p, miR-32-5p, miR-126-3p, miR-126-5p, miR-133a-3p, miR-133b, miR-199b-5p, miR-210-3p, miR-221-5p, miR-324-5p, miR-363-3p, and let-7c-5p) were calculated from the obtained sequence data. These results are shown in FIGs.

22A to 22E. As the statistical processing in the graph, a multiple comparison test of Tukey was performed after Two-way ANOVA test.

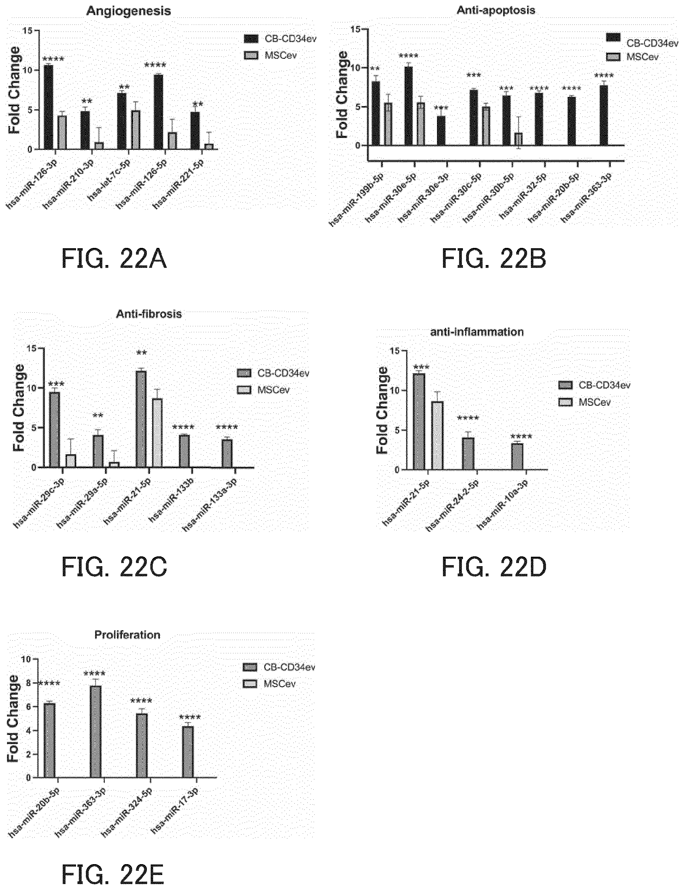

FIGs. 22A to 22E are graphs each showing the expression level of miRNA. FIG. 22A shows the expression level of miRNA having angiogenesis promoting activity, FIG. 22B shows the expression level of miRNA having anti-apoptotic activity, FIG. 22C shows the expression level of miRNA having anti-fibrotic activity, FIG. 22D shows the expression level of miRNA having anti-inflammatory activity, and FIG. 22E shows the expression level of miRNA having cell growth promoting activity. The horizontal axis indicates the type of miRNA, and the vertical axis indicates the relative expression level. As shown in 22A to 22E, CB-CD34ev had significantly high expression levels of miRNAs (miR-126-3p, miR-210-3p, let-7c-5p, miR-126-5p, and miR-221-5p) having angiogenesis promoting activity, miRNAs (miR-199b-5p, miR-30e-5p, miR-30e-3p, miR-30c-5p, miR-30b-5p, miR-32-5p, miR-20b-5p, and miR-363-3p) having anti-apoptotic activity, miRNAs (miR-29c-3p, miR-29a-5p, miR-21-5p, miR-133b, and miR-133a-3p) having anti-fibrotic activity, miRNAs (miR-21-5p, miR-24-2-5p, and miR-10a-3p) having anti-inflammatory activity, and miRNAs (miR-20b-5p, miR-363-3p, miR-324-5p, and miR-17-3p) having cell growth promoting activity as compared to those of MSCev. From these results, it was found that CB-CD34ev contains miRNA having angiogenesis promoting activity, miRNA having anti-apoptotic activity, miRNA having anti-fibrotic activity, and miRNA having cell growth promoting activity.

From the above, it was suggested that the umbilical cord blood-derived mononuclear cells or CD34 positive progenitor cells being cultured with various factors have angiogenesis promoting activity, inflammation suppressing activity, apoptosis suppressing activity, vascular endothelial cell proliferating activity, and fibrosis suppressing activity.

While the present invention has been described above with reference to illustrative embodiments and examples, the present invention is by no means limited thereto. Various changes and modifications that may become apparent to those skilled in the art may be made in the configuration and specifics of the present invention without departing from the scope of the present invention.

This application claims priority from Japanese Patent Application No. 2021-81426 filed on May 13, 2021. The entire subject matter of the Japanese Patent Application is incorporated herein by reference.

<Supplementary Notes>

Some or all of the above-described embodiments and examples may be described as the following supplementary notes, but they are not limited thereto.

<Composition>

(Supplementary Note 1)

A composition including:
an extracellular vesicle of a regenerative cell population induced from a biological sample-derived mononuclear cell or a culture thereof.

(Supplementary Note 2)

The composition according to Supplementary Note 1, wherein
the extracellular vesicle includes at least one miRNA selected from the group consisting of miR-15b-5p, miR-29b-3p, miR-29c-3p, miR-92a-2-5p, miR-126-3p, miR-126-5p, miR-133a-3p, miR-133b, miR-146a-3p, miR-146b-5p, miR-150-5p, miR-181b-3p, miR-195-3p, miR-195-5p, miR-200b-5p, miR-302a-5p, and miR-142-3p.

(Supplementary Note 3)

The composition according to Supplementary Note 1 or 2, wherein
the extracellular vesicle further includes at least one miRNA selected from the group consisting of miR-10a-3p, miR-17-3p, miR-20b-5p, miR-21-5p, miR-24-2-5p, miR-29a-5p, miR-30b-5p, miR-30c-5p, miR-30e-3p, miR-30e-5p, miR-32-5p, miR-92a-3p, miR-103a-2-5p, miR-144-5p, miR-148a-3p, miR-199b-5p, miR-200a-3p, miR-205-5p, miR-210-3p, miR-221-5p, miR-324-5p, miR-363-3p, miR-373-3p, miR-509-3p, miR-633, and let-7c-5p.

(Supplementary Note 4)

The composition according to any one of Supplementary Notes 1 to 3, wherein
the extracellular vesicle is positive for at least one extracellular vesicle marker selected from the group consisting of CD9 and CD63.

(Supplementary Note 5)

The composition according to any one of Supplementary Notes 1 to 4, wherein
the mean diameter of the extracellular vesicle is 10 to 500 nm.

(Supplementary Note 6)

The composition according to any one of Supplementary Notes 1 to 5, wherein the composition has a vascular endothelial cell growth promoting activity.

(Supplementary Note 7)

The composition according to any one of Supplementary Notes 1 to 6, wherein the composition has an angiogenesis promoting activity.

(Supplementary Note 8)

The composition according to any one of Supplementary Notes 1 to 7, wherein the composition has a fibrosis suppressing activity.

(Supplementary Note 9)

The composition according to any one of Supplementary Notes 1 to 8, wherein the composition has a bioactivity with a reduced transplant rejection.

(Supplementary Note 10)

The composition according to any one of Supplementary Notes 1 to 9, wherein
the extracellular vesicle is an extracellular vesicle extracted or isolated from the regenerative cell population or the culture thereof.

(Supplementary Note 11)

The composition according to any one of Supplementary Notes 1 to 10, wherein
the regenerative cell population is induced by culturing the mononuclear cells in the presence of at least one factor selected from the group consisting of a stem cell factor, interleukin-6, FMS like tyrosine kinase-3 ligand, thrombopoietin, and a vascular endothelial cell growth factor.

(Supplementary Note 12)

The composition according to any one of Supplementary Notes 1 to 11, wherein
the regenerative cell population is induced without sorting at least one cell selected from the group consisting of a CD34 positive cell and/or a CD133 positive cell.

(Supplementary Note 13)

The composition according to any one of Supplementary Notes 1 to 12, wherein
the regenerative cell population includes endothelial progenitor cells and anti-inflammatory macrophages.

(Supplementary Note 14)

The composition according to Supplementary Note 13, wherein
the endothelial progenitor cell is a differentiated EPC colony-forming cell.

(Supplementary Note 15)

The composition according to Supplementary Note 13 or 14, wherein
the anti-inflammatory macrophage is a M2 macrophage.

(Supplementary Note 16)

The composition according to any one of Supplementary Notes 1 to 15, wherein
the biological sample is at least one tissue selected from the group consisting of blood and bone marrow.

(Supplementary Note 17)

The composition according to any one of Supplementary Notes 1 to 16, wherein
the biological sample is at least one tissue selected from the group consisting of peripheral blood, and umbilical cord blood.

<Composition for Treating Ischemic Heart Disease>

(Supplementary Note 18)

A composition for use in treating ischemic heart disease, wherein
the composition is the composition according to any one of Supplementary Notes 1 to 17.

(Supplementary Note 19)

The composition according to Supplementary Note 18, wherein
the ischemic heart disease is myocardial infarction.

<Composition for Promoting Vascular Endothelial Cell Growth>

(Supplementary Note 20)

A composition for use in promoting vascular endothelial cell growth, wherein
the composition is the composition according to any one of Supplementary Notes 1 to 17.

<Composition for Inducing Angiogenesis >

(Supplementary Note 21)

A composition for use in inducing angiogenesis, wherein
the composition is the composition according to any one of Supplementary Notes 1 to 17.

<Composition for Suppressing Fibrosis>

(Supplementary Note 22)

A composition for use in suppressing fibrosis, wherein
the composition is the composition according to any one of Supplementary Notes 1 to 17.

<Method for Treating Ischemic Heart Disease>

(Supplementary Note 23)

A method for treating a patient with ischemic heart disease including:
administering the composition according to any one of Supplementary Notes 1 to 17 to the patient with ischemic heart disease.

(Supplementary Note 24)

The method according to Supplementary Note 23, wherein
the ischemic heart disease is myocardial infarction, peripheral arterial disease, myocardial ischemia reperfusion injury, or severe leg ischemia.

(Supplementary Note 25)

A method for promoting vascular endothelial cell growth, including:

promoting vascular endothelial cell growth by bringing vascular endothelial cells into contact with a composition, wherein
the composition is the composition according to any one of Supplementary Notes 1 to 17.

<Method for Inducing Angiogenesis>

(Supplementary Note 26)

A method for inducing angiogenesis, using the composition according to any one of Supplementary Notes 1 to 17.

(Supplementary Note 27)

The method according to Supplementary Note 26, including:
administering the composition to a subject to be induced with angiogenesis.

(Supplementary Note 28)

The method according to Supplementary Note 27 or 28, wherein

the subject to be induced with angiogenesis is a patient with ischemic heart disease, and
the induction of angiogenesis is induction of angiogenesis in a heart of a patient with ischemic heart disease.

<Method for Suppressing Fibrosis>

(Supplementary Note 29)

A method for suppressing fibrosis, using the composition according to any one of Supplementary Notes 1 to 17.

(Supplementary Note 30)

The method according to Supplementary Note 29, including:
administering the composition to a subject to be suppressed with fibrosis.

(Supplementary Note 31)

The method according to Supplementary Note 29 or 30, wherein

the subject to be suppressed with fibrosis is a patient with ischemic heart disease, and
the suppression of fibrosis is suppression of fibrosis in a heart of a patient with ischemic heart disease.

<Use>

(Supplementary Note 32)

A composition for use in treating ischemic heart disease, wherein
the composition is the composition according to any one of Supplementary Notes 1 to 17.

(Supplementary Note 33)

A composition for use in promoting vascular endothelial cell growth, wherein
the composition is the composition according to any one of Supplementary Notes 1 to 17.

(Supplementary Note 34)

A composition for use in inducing angiogenesis, wherein
the composition is the composition according to any one of Supplementary Notes 1 to 17.

(Supplementary Note 35)

A composition for use in suppressing fibrosis, wherein
the composition is the composition according to any one of Supplementary Notes 1 to 17.

Industrial Applicability

As described above, the transplant rejection of the extracellular vesicle or composition of the present invention is suppressed, and the extracellular vesicle or composition of the present invention is derived from the regenerative cell population. Thus, the extracellular vesicle or composition of the present invention has bioactivities such as vascular endothelial cell growth promoting activity and angiogenesis promoting activity. Thus, the present invention can be suitably used, for example, for treating ischemic heart disease or for improving the prognosis. Therefore, the present invention is extremely useful, for example, in the pharmaceutical field and the like.

**Claims**

1. A composition comprising:
   an extracellular vesicle of a regenerative cell population induced from a biological sample-derived mononuclear cell or a culture thereof.

2. The composition according to claim 1, wherein
   the extracellular vesicle comprises at least one miRNA selected from the group consisting of miR-15b-5p, miR-29b-3p, miR-29c-3p, miR-92a-2-5p, miR-126-3p, miR-126-5p, miR-133a-3p, miR-133b, miR-146a-3p, miR-146b-5p, miR-150-5p, miR-181b-3p, miR-195-3p, miR-195-5p, miR-200b-5p, miR-302a-5p, and miR-142-3p.

3. The composition according to claim 2, wherein
   the extracellular vesicle further comprises at least one miRNA selected from the group consisting of miR-10a-3p, miR-17-3p, miR-20b-5p, miR-21-5p, miR-24-2-5p, miR-29a-5p, miR-30b-5p, miR-30c-5p, miR-30e-3p, miR-30e-5p, miR-32-5p, miR-92a-3p, miR-103a-2-5p, miR-144-5p, miR-148a-3p, miR-199b-5p, miR-200a-3p, miR-205-5p, miR-210-3p, miR-221-5p, miR-324-5p, miR-363-3p, miR-373-3p, miR-509-3p, miR-633, and let-7c-5p.

4. The composition according to any one of claims 1 to 3, wherein
   the extracellular vesicle is positive for at least one extracellular vesicle marker selected from the group consisting of CD9 and CD63.

5. The composition according to any one of claims 1 to 4, wherein
   a mean diameter of the extracellular vesicle is in a range from 10 to 500 nm.

6. The composition according to any one of claims 1 to 5, wherein the composition has a vascular endothelial cell growth promoting activity.

7. The composition according to any one of claims 1 to 6, wherein the composition has a angiogenesis promoting activity.

8. The composition according to any one of claims 1 to 7, wherein the composition has a fibrosis suppressing activity.

9. The composition according to any one of claims 1 to 8, wherein the composition has a bioactivity with reduced transplant rejection.

10. The composition according to any one of claims 1 to 9, wherein
    the extracellular vesicle is an extracellular vesicle extracted or isolated from the regenerative cell population or the culture thereof.

**11.** The composition according to any one of claims 1 to 10, wherein
the regenerative cell population is induced by culturing the mononuclear cells in the presence of at least one factor selected from the group consisting of a stem cell factor, interleukin-6, FMS like tyrosine kinase-3 ligand, thrombopoietin, and a vascular endothelial cell growth factor.

**12.** The composition according to any one of claims 1 to 11, wherein
the regenerative cell population is induced without sorting at least one cell selected from the group consisting of a CD34 positive cell and a CD133 positive cell.

**13.** The composition according to any one of claims 1 to 12, wherein
the regenerative cell population comprises endothelial progenitor cells and anti-inflammatory macrophages.

**14.** The composition according to claim 13, wherein
the endothelial progenitor cell is a differentiated EPC colony-forming cell.

**15.** The composition according to claim 12 or 14, wherein
the anti-inflammatory macrophage is a M2 macrophage.

**16.** The composition according to any one of claims 1 to 15, wherein
the biological sample is at least one tissue selected from the group consisting of blood and bone marrow.

**17.** The composition according to any one of claims 1 to 16, wherein
the biological sample is at least one tissue selected from the group consisting of peripheral blood and umbilical cord blood.

**18.** A composition for use in treating ischemic heart disease, wherein
the composition is the composition according to any one of claims 1 to 17.

**19.** The composition according to claim 18, wherein
the ischemic heart disease is myocardial infarction, peripheral arterial disease, myocardial ischemia reperfusion injury, or severe leg ischemia.

**20.** A composition for use in promoting vascular endothelial cell growth, wherein
the composition is the composition according to any one of claims 1 to 17.

**21.** A composition for use in inducing angiogenesis, wherein
the composition is the composition according to any one of claims 1 to 17.

**22.** A composition for use in suppressing fibrosis, wherein
the composition is the composition according to any one of claims 1 to 17.

FIG. 1A

FIG. 1B

FIG. 1C

FIG. 2

FIG. 3A

FIG. 3B

FIG. 4

FIG. 5A

FIG. 5B

FIG. 6

30

Control

## FIG. 7A

MSCev

## FIG. 7B

RACev

## FIG. 7C

Time before and after onset of
ischemia-reperfusion injury

## FIG. 7D

Time before and after onset of
ischemia-reperfusion injury

## FIG. 7E

FIG. 8A

FIG. 8B

## Interstitial fibrosis

FIG. 9A

FIG. 9B

## anti-fibrosis miR

FIG. 10

FIG. 11A

FIG. 11B

FIG. 12

FIG. 13

FIG. 14A

FIG. 14B

**FIG. 15A**

**FIG. 15B**

FIG. 16

FIG. 17

FIG. 18

PBMNC QQ cells

FIG. 19A

CBMNC QQ cells

FIG. 19B

3 medium PBMNCQQ^R5 2011

FIG. 20A

CBMNC pre & post QQ

FIG. 20B

FIG. 21A

FIG. 21B

FIG. 21C

FIG. 21D

FIG. 21E

FIG. 22A

FIG. 22B

FIG. 22C

FIG. 22D

FIG. 22E

## INTERNATIONAL SEARCH REPORT

| | International application No. |
|---|---|
| | **PCT/JP2022/020222** |

### A. CLASSIFICATION OF SUBJECT MATTER

*A61K 35/15*(2015.01)i; *A61K 35/51*(2015.01)i; *A61P 9/10*(2006.01)i; *A61P 9/14*(2006.01)i; *A61P 43/00*(2006.01)i; *C12N 5/071*(2010.01)n
FI: A61K35/15 Z; A61K35/51; A61P9/10; A61P9/14; A61P43/00 105; C12N5/071

According to International Patent Classification (IPC) or to both national classification and IPC

### B. FIELDS SEARCHED

Minimum documentation searched (classification system followed by classification symbols)

A61K35/15; A61K35/51; A61P9/10; A61P9/14; A61P43/00; C12N5/071

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | CN 111249293 A (THE FIRST HOSPITAL OF HUNAN UNIVERSITY OF CHINESE MEDICINE) 09 June 2020 (2020-06-09)<br>claims 7, 8, table 1, paragraphs [0002]-[0004], [0006], [0020], [0036], [0072]-[0073], [0082], [0113]-[0116] | 1-7, 10-12, 16-21 |
| Y | | 1-22 |
| Y | SALYBEKOV, A. A. et al. Regeneration-associated cells improve recovery from myocardial infarction through enhanced vasculogenesis, anti-inflammation, and cardiomyogenesis. PLOS ONE. 13(11), e0203244<br>Abstract, p. 3, "PBMNC isolation and QQ culture", p. 7, lines 20-25, p. 15, lines 6-9, fig. 1, 9 | 1-22 |
| Y | US 2020/0390822 A1 (MUSC FOUNDATION FOR RESEARCH DEVELOPMENT) 17 December 2020 (2020-12-17)<br>paragraphs [0006], [0008], claim 1 | 1-22 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 June 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
|---|
| **PCT/JP2022/020222** |

### C.    DOCUMENTS CONSIDERED TO BE RELEVANT

| Category* | Citation of document, with indication, where appropriate, of the relevant passages | Relevant to claim No. |
|---|---|---|
| P, X | SALYBEKOV, A. A. et al. Extracellular Vesicles Derived From Regeneration Associated Cells Preserve Heart Function After Ischemia-Induced Injury. Frontiers in Cardiovascular Medicine. 20 October 2021, 8, 754254 <br> abstract | 1-22 |

Form PCT/ISA/210 (second sheet) (January 2015)

INTERNATIONAL SEARCH REPORT
Information on patent family members

International application No.

**PCT/JP2022/020222**

| Patent document cited in search report | Publication date (day/month/year) | Patent family member(s) | Publication date (day/month/year) |
|---|---|---|---|
| CN 111249293 A | 09 June 2020 | (Family: none) | |
| US 2020/0390822 A1 | 17 December 2020 | WO 2019/168977 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011529957 A
- WO 2014051154 A
- JP 2021081426 A

### Non-patent literature cited in the description

- **MASUDA H. et al.** *Circulation Research,* 2011, vol. 109, 20-37
- **ASAHARA et al.** *Science,* 1997, vol. 275, 964-7
- **AMANKELDI.** *PLoSONE,* 2019, vol. 14 (3), e0205477
- **THERY C.** *J.Extracell Vesicles,* 2018, vol. 7, 1535750
- **SALYBEKOV.** *PLoSONE,* 2018, vol. 13 (11), e0203244
- **SALYBEKOV AA.** *PLoS One,* 2019, vol. 14, e0205477
- **MASUDA H. et al.** *Circulation research,* 2011, vol. 109, 20-37